# EUROPEAN PATENT APPLICATION

(11) **EP 1 486 506 A1**
(43) Date of publication of application: **15.12.2004**
(21) Application number: 03291377.4
(22) Date of filing: 10.06.2003
(51) Int. Cl.: C07K 14/035, C12N 9/12

(54) **Herpes simplex virus US11 protein and HIPK2 protein complexes and uses thereof**

(71) Applicant: INSTITUT NATIONAL DE LA SANTE ET DE LA RECHERCHE MEDICALE (INSERM), 75013 Paris (FR)
(72) Inventor: Diaz, Jean-Jaques, 69200 Venissieux (FR); Giraud, Stéphane, 38080 L'isle D'abeau (FR)
(74) Representative: Bernasconi, Jean Raymond

(57) **Abstract**

The present invention relates to the inhibition of cellular apoptosis by the US11 protein from a herpes simplex virus, through binding of US11 to the apoptosis inducer HIPK2. The invention more particularly relates to a US11/HIPK2 complex and provides methods and compositions for the treatment of a herpes simplex virus infection.

SEQUENCE LISTING

<110>  INSERM

<120>  Human herpes simplex virus US11 protein and HIPK2 protein complexes, and
uses thereof

<130>  BET 03P0116

<160>  27

<170>  PatentIn version 3.1

<210>  1

<211>  4000

<212>  DNA

<213>  Homo sapiens

<220>

<221>  CDS

<222>  (109)..(3075)

<223>

<400>  1
cggccgcttt tttctcaaga tggcagattc ccactgaggc tgagggggcc gagctcgcgc      60

gccgcgttcc cttctccgtt gccatgaacc gcggacaccc cggccccg atg gcc ccc      117
                                                     Met Ala Pro
                                                     1

gtg tac gaa ggt atg gcc tca cat gtg caa gtt ttc tcc cct cac acc      165
Val Tyr Glu Gly Met Ala Ser His Val Gln Val Phe Ser Pro His Thr
    5                   10                  15

ctt caa tca agt gcc ttc tgt agt gtg aag aaa cta aaa gta gag cca      213
Leu Gln Ser Ser Ala Phe Cys Ser Val Lys Lys Leu Lys Val Glu Pro
20                  25                  30                  35

agt tcc aac tgg gac atg act ggg tac ggc tcc cac agc aaa gtg tac      261

Ser Ser Asn Trp Asp Met Thr Gly Tyr Gly Ser His Ser Lys Val Tyr
                40                  45                  50

agc cag agc aag aac ata cca cct tct cag cca gcc tcc aca acc gtc       309
Ser Gln Ser Lys Asn Ile Pro Pro Ser Gln Pro Ala Ser Thr Thr Val
            55                  60                  65

agc acc tcc ttg ccg gtc cca aac cca agc cta cct tac gag cag acc       357
Ser Thr Ser Leu Pro Val Pro Asn Pro Ser Leu Pro Tyr Glu Gln Thr
        70                  75                  80

atc gtc ttc cca gga agc acc ggg cac atc gtg gtc acc tca gca agc       405
Ile Val Phe Pro Gly Ser Thr Gly His Ile Val Val Thr Ser Ala Ser
    85                  90                  95

agc act tct gtc acc ggg caa gtc ctc ggc gga cca cac aac cta atg       453
Ser Thr Ser Val Thr Gly Gln Val Leu Gly Gly Pro His Asn Leu Met
100                 105                 110                 115

cgt cga agc act gtg agc ctc ctt gat acc tac caa aaa tgt gga ctc       501
Arg Arg Ser Thr Val Ser Leu Leu Asp Thr Tyr Gln Lys Cys Gly Leu
                120                 125                 130

aag cgt aag agc gag gag atc gag aac aca agc agc gtg cag atc atc       549
Lys Arg Lys Ser Glu Glu Ile Glu Asn Thr Ser Ser Val Gln Ile Ile
            135                 140                 145

gag gag cat cca ccc atg att cag aat aat gca agc ggg gcc act gtc       597
Glu Glu His Pro Pro Met Ile Gln Asn Asn Ala Ser Gly Ala Thr Val
        150                 155                 160

gcc act gcc acc acg tct act gcc acc tcc aaa aac agc ggc tcc aac       645
Ala Thr Ala Thr Thr Ser Thr Ala Thr Ser Lys Asn Ser Gly Ser Asn
    165                 170                 175

agc gag ggc gac tat cag ctg gtg cag cat gag gtg ctg tgc tcc atg       693
Ser Glu Gly Asp Tyr Gln Leu Val Gln His Glu Val Leu Cys Ser Met
180                 185                 190                 195

acc aac acc tac gag gtc tta gag ttc ttg ggc cga ggg acg ttt gga       741
Thr Asn Thr Tyr Glu Val Leu Glu Phe Leu Gly Arg Gly Thr Phe Gly
                200                 205                 210

caa gtg gtc aag tgc tgg aaa cgg ggc acc aat gag atc gta gcc atc       789
Gln Val Val Lys Cys Trp Lys Arg Gly Thr Asn Glu Ile Val Ala Ile
            215                 220                 225

aag atc ctg aag aac cgc cca tcc tat gcc cga caa ggt cag att gaa       837
Lys Ile Leu Lys Asn Arg Pro Ser Tyr Ala Arg Gln Gly Gln Ile Glu
        230                 235                 240

gtg agc atc ctg gcc cgg ttg agc acg gag agt gcc gat gac tat aac       885
Val Ser Ile Leu Ala Arg Leu Ser Thr Glu Ser Ala Asp Asp Tyr Asn
    245                 250                 255

ttc gtc cgg gcc tac gaa tgc ttc cag cac aag aac cac acg tgc ttg       933
Phe Val Arg Ala Tyr Glu Cys Phe Gln His Lys Asn His Thr Cys Leu
260                 265                 270                 275

gtc ttc gag atg ttg gag cag aac ctc tat gac ttt ctg aag caa aac       981
Val Phe Glu Met Leu Glu Gln Asn Leu Tyr Asp Phe Leu Lys Gln Asn

280                 285                 290

aag ttt agc ccc ttg ccc ctc aaa tac att cgc cca gtt ctc cag cag      1029
Lys Phe Ser Pro Leu Pro Leu Lys Tyr Ile Arg Pro Val Leu Gln Gln
            295                 300                 305

gta gcc aca gcc ctg atg aaa ctc aaa agc cta ggt ctt atc cac gct      1077
Val Ala Thr Ala Leu Met Lys Leu Lys Ser Leu Gly Leu Ile His Ala
        310                 315                 320

gac ctc aaa cca gaa aac atc atg ctg gtg gat cca tct aga caa cca      1125
Asp Leu Lys Pro Glu Asn Ile Met Leu Val Asp Pro Ser Arg Gln Pro
    325                 330                 335

tac aga gtc aag gtc atc gac ttt ggt tca gcc agc cac gtc tcc aag      1173
Tyr Arg Val Lys Val Ile Asp Phe Gly Ser Ala Ser His Val Ser Lys
340                 345                 350                 355

gct gtg tgc tcc acc tac ttg cag tcc aga tat tac agg gcc cct gag      1221
Ala Val Cys Ser Thr Tyr Leu Gln Ser Arg Tyr Tyr Arg Ala Pro Glu
                360                 365                 370

atc atc ctt ggt tta cca ttt tgt gag gca att gac atg tgg tcc ctg      1269
Ile Ile Leu Gly Leu Pro Phe Cys Glu Ala Ile Asp Met Trp Ser Leu
            375                 380                 385

ggc tgt gtt att gca gaa ttg ttc ctg ggt tgg ccg tta tat cca gga      1317
Gly Cys Val Ile Ala Glu Leu Phe Leu Gly Trp Pro Leu Tyr Pro Gly
        390                 395                 400

gct tcg gag tat gat cag att cgg tat att tca caa aca cag ggt ttg      1365
Ala Ser Glu Tyr Asp Gln Ile Arg Tyr Ile Ser Gln Thr Gln Gly Leu
    405                 410                 415

cct gct gaa tat tta tta agc gcc ggg aca aag aca act agg ttt ttc      1413
Pro Ala Glu Tyr Leu Leu Ser Ala Gly Thr Lys Thr Thr Arg Phe Phe
420                 425                 430                 435

aac cgt gac acg gac tca cca tat cct ttg tgg aga ctg aag aca cca      1461
Asn Arg Asp Thr Asp Ser Pro Tyr Pro Leu Trp Arg Leu Lys Thr Pro
                440                 445                 450

gat gac cat gaa gca gag aca ggg att aag tca aaa gaa gca aga aag      1509
Asp Asp His Glu Ala Glu Thr Gly Ile Lys Ser Lys Glu Ala Arg Lys
            455                 460                 465

tac att ttc aac tgt tta gat gat atg gcc cag gtg aac atg acg aca      1557
Tyr Ile Phe Asn Cys Leu Asp Asp Met Ala Gln Val Asn Met Thr Thr
        470                 475                 480

gat ttg gaa ggg agc gac atg ttg gta gaa aag gct gac cgg cgg gag      1605
Asp Leu Glu Gly Ser Asp Met Leu Val Glu Lys Ala Asp Arg Arg Glu
    485                 490                 495

ttc att gac ctg ttg aag aag atg ctg acc att gat gct gac aag aga      1653
Phe Ile Asp Leu Leu Lys Lys Met Leu Thr Ile Asp Ala Asp Lys Arg
500                 505                 510                 515

atc act cca atc gaa acc ctg aac cat ccc ttt gtc acc atg aca cac      1701
Ile Thr Pro Ile Glu Thr Leu Asn His Pro Phe Val Thr Met Thr His
                520                 525                 530

tta ctc gat ttt ccc cac agc aca cac gtc aaa tca tgt ttc cag aac     1749
Leu Leu Asp Phe Pro His Ser Thr His Val Lys Ser Cys Phe Gln Asn
            535                 540                 545

atg gag atc tgc aag cgt cgg gtg aat atg tat gac acg gtg aac cag     1797
Met Glu Ile Cys Lys Arg Arg Val Asn Met Tyr Asp Thr Val Asn Gln
        550                 555                 560

agc aaa acc cct ttc atc acg cac gtg gcc ccc agc acg tcc acc aac     1845
Ser Lys Thr Pro Phe Ile Thr His Val Ala Pro Ser Thr Ser Thr Asn
    565                 570                 575

ctg acc atg acc ttt aac aac cag ctg acc act gtc cac aac cag gct     1893
Leu Thr Met Thr Phe Asn Asn Gln Leu Thr Thr Val His Asn Gln Ala
580                 585                 590                 595

ccc tcc tct acc agt gcc act att tcc tta gcc aat ccc gaa gtc tcc     1941
Pro Ser Ser Thr Ser Ala Thr Ile Ser Leu Ala Asn Pro Glu Val Ser
                600                 605                 610

ata cta aac tac cca tct aca ctc tac cag ccc tca gcg gca tcc atg     1989
Ile Leu Asn Tyr Pro Ser Thr Leu Tyr Gln Pro Ser Ala Ala Ser Met
            615                 620                 625

gct gca gtg gcc cag cgg agc atg ccc ctg cag aca gga aca gcc cag     2037
Ala Ala Val Ala Gln Arg Ser Met Pro Leu Gln Thr Gly Thr Ala Gln
        630                 635                 640

att tgt gcc cgg cct gac ccg ttc cag caa gct ctc atc gtg tgt ccc     2085
Ile Cys Ala Arg Pro Asp Pro Phe Gln Gln Ala Leu Ile Val Cys Pro
    645                 650                 655

ccc ggc ttc caa ggc ttg cag gcc tct ccc tct aag cac gct ggc tac     2133
Pro Gly Phe Gln Gly Leu Gln Ala Ser Pro Ser Lys His Ala Gly Tyr
660                 665                 670                 675

tcg gtg cga atg gaa aat gca gtt ccc atc gtc act caa gcc cca gga     2181
Ser Val Arg Met Glu Asn Ala Val Pro Ile Val Thr Gln Ala Pro Gly
                680                 685                 690

gct cag cct ctt cag atc caa cca ggt ctg ctt gcc cag cag gct tgg     2229
Ala Gln Pro Leu Gln Ile Gln Pro Gly Leu Leu Ala Gln Gln Ala Trp
            695                 700                 705

cca agt ggg acc cag cag atc ctg ctt ccc cca gca tgg cag caa ctg     2277
Pro Ser Gly Thr Gln Gln Ile Leu Leu Pro Pro Ala Trp Gln Gln Leu
        710                 715                 720

act gga gtg gcc acc cac aca tca gtg cag cat gcc acc gtg att ccc     2325
Thr Gly Val Ala Thr His Thr Ser Val Gln His Ala Thr Val Ile Pro
    725                 730                 735

gag acc atg gca ggc acc cag cag ctg gcg gac tgg aga aat acg cat     2373
Glu Thr Met Ala Gly Thr Gln Gln Leu Ala Asp Trp Arg Asn Thr His
740                 745                 750                 755

gct cac gga agc cat tat aat ccc atc atg cag cag cct gca cta ttg     2421
Ala His Gly Ser His Tyr Asn Pro Ile Met Gln Gln Pro Ala Leu Leu
                760                 765                 770

acc ggt cat gtg acc ctt cca gca gca cag ccc tta aat gtg ggt gtg     2469
Thr Gly His Val Thr Leu Pro Ala Ala Gln Pro Leu Asn Val Gly Val
            775                 780                 785

gcc cac gtg atg cgg cag cag cca acc agc acc acc tcc tcc cgg aag     2517
Ala His Val Met Arg Gln Gln Pro Thr Ser Thr Thr Ser Ser Arg Lys
        790                 795                 800

agt aag cag cac cag tca tct gtg aga aat gtc tcc acc tgt gag gtg     2565
Ser Lys Gln His Gln Ser Ser Val Arg Asn Val Ser Thr Cys Glu Val
    805                 810                 815

tcc tcc tct cag gcc atc agc tcc cca cag cga tcc aag cgt gtc aag     2613
Ser Ser Ser Gln Ala Ile Ser Ser Pro Gln Arg Ser Lys Arg Val Lys
820                 825                 830                 835

gag aac aca cct ccc cgc tgt gcc atg gtg cac agt agc ccg gcc tgc     2661
Glu Asn Thr Pro Pro Arg Cys Ala Met Val His Ser Ser Pro Ala Cys
                840                 845                 850

agc acc tcg gtc acc tgt ggg tgg ggc gac gtg gcc tcc agc acc acc     2709
Ser Thr Ser Val Thr Cys Gly Trp Gly Asp Val Ala Ser Ser Thr Thr
            855                 860                 865

cgg gaa cgg cag cgg cag aca att gtc att ccc gac act ccc agc ccc     2757
Arg Glu Arg Gln Arg Gln Thr Ile Val Ile Pro Asp Thr Pro Ser Pro
        870                 875                 880

acg gtc agc gtc atc acc atc agc agt gac acg gac gag gag gag gaa     2805
Thr Val Ser Val Ile Thr Ile Ser Ser Asp Thr Asp Glu Glu Glu Glu
    885                 890                 895

cag aaa cac gcc ccc acc agc act gtc tcc aag caa aga aaa aac gtc     2853
Gln Lys His Ala Pro Thr Ser Thr Val Ser Lys Gln Arg Lys Asn Val
900                 905                 910                 915

atc agc tgt gtc aca gtc cac gac tcc ccc tac tcc gac tcc tcc agc     2901
Ile Ser Cys Val Thr Val His Asp Ser Pro Tyr Ser Asp Ser Ser Ser
                920                 925                 930

aac acc agc ccc tac tcc gtg cag cag cgt gct ggg cac aac aat gcc     2949
Asn Thr Ser Pro Tyr Ser Val Gln Gln Arg Ala Gly His Asn Asn Ala
            935                 940                 945

aat gcc ttt gac acc aag ggg agc ctg gag aat cac tgc acg ggg aac     2997
Asn Ala Phe Asp Thr Lys Gly Ser Leu Glu Asn His Cys Thr Gly Asn
        950                 955                 960

ccc cga acc atc atc gtg cca ccc ctg aaa acc cag gcc agc gaa gta     3045
Pro Arg Thr Ile Ile Val Pro Pro Leu Lys Thr Gln Ala Ser Glu Val
    965                 970                 975

ttg gtg gag tgt gat agc ctg gtg cca gtc aacaccagtc accactcgtc     3095
Leu Val Glu Cys Asp Ser Leu Val Pro Val
980                 985

ctcctacaag tccaagtcct ccagcaacgt gacctccacc agcggtcact cttcagggag     3155

ctcatctgga gccatcacct accggcagca gcggccgggc ccccacttcc agcagcagca     3215

gccactcaat ctcagccagg ctcagcagca catcaccacg gaccgcactg ggagccaccg     3275

aaggcagcag gcctacatca ctcccaccat ggcccaggct ccgtactcct tcccgcacaa 3335

cagccccagc cacggcactg tgcacccgca tctggctgca gccgctgccg ctgcccacct 3395

ccccacccag ccccacctct acacctacac tgcgccggcg gccctgggct ccaccggcac 3455

cgtggcccac ctggtggcct cgcaaggctc tgcgcgccac accgtgcagc acactgccta 3515

cccagccagc atcgtccacc aggtccccgt gagcatgggc ccccgggtcc tgccctcgcc 3575

caccatccac ccgagtcagt atccagccca atttgcccac cagacctaca tcagcgcctc 3635

gccagcctcc accgtctaca ctggataccc actgagcccc gccaaggtca accagtaccc 3695

ttacatataa acactggagg ggagggaggg agggagggag ggagagaatg gcccgaggga 3755

ggagggagag aaggagggag gcgctcctgg gaccgtgggc gctggccttt tatactgaag 3815

atgccgcaca caaacaatgc aaacggggca ggggcggggg gggggggggc agagggcagg 3875

gggacgggtc gggacaccag tgaaacttga accgggaagt gggaggacgt agagcagaga 3935

agagaacatt tttaaaagga agggattaaa gagggtggga aatctatggt ttttatttta 3995

aaaaa 4000

<210> 2

<211> 989

<212> PRT

<213> Homo sapiens

<400> 2

Met Ala Pro Val Tyr Glu Gly Met Ala Ser His Val Gln Val Phe Ser
1               5                   10                  15

Pro His Thr Leu Gln Ser Ser Ala Phe Cys Ser Val Lys Lys Leu Lys
            20                  25                  30

Val Glu Pro Ser Ser Asn Trp Asp Met Thr Gly Tyr Gly Ser His Ser
        35                  40                  45

Lys Val Tyr Ser Gln Ser Lys Asn Ile Pro Pro Ser Gln Pro Ala Ser
    50                  55                  60

Thr Thr Val Ser Thr Ser Leu Pro Val Pro Asn Pro Ser Leu Pro Tyr
65                  70                  75                  80

Glu Gln Thr Ile Val Phe Pro Gly Ser Thr Gly His Ile Val Val Thr

                    85                  90                  95

Ser Ala Ser Ser Thr Ser Val Thr Gly Gln Val Leu Gly Gly Pro His
            100                 105                 110

Asn Leu Met Arg Arg Ser Thr Val Ser Leu Leu Asp Thr Tyr Gln Lys
        115                 120                 125

Cys Gly Leu Lys Arg Lys Ser Glu Glu Ile Glu Asn Thr Ser Ser Val
    130                 135                 140

Gln Ile Ile Glu Glu His Pro Pro Met Ile Gln Asn Asn Ala Ser Gly
145                 150                 155                 160

Ala Thr Val Ala Thr Ala Thr Thr Ser Thr Ala Thr Ser Lys Asn Ser
                165                 170                 175

Gly Ser Asn Ser Glu Gly Asp Tyr Gln Leu Val Gln His Glu Val Leu
            180                 185                 190

Cys Ser Met Thr Asn Thr Tyr Glu Val Leu Glu Phe Leu Gly Arg Gly
        195                 200                 205

Thr Phe Gly Gln Val Val Lys Cys Trp Lys Arg Gly Thr Asn Glu Ile
    210                 215                 220

Val Ala Ile Lys Ile Leu Lys Asn Arg Pro Ser Tyr Ala Arg Gln Gly
225                 230                 235                 240

Gln Ile Glu Val Ser Ile Leu Ala Arg Leu Ser Thr Glu Ser Ala Asp
                245                 250                 255

Asp Tyr Asn Phe Val Arg Ala Tyr Glu Cys Phe Gln His Lys Asn His
            260                 265                 270

Thr Cys Leu Val Phe Glu Met Leu Glu Gln Asn Leu Tyr Asp Phe Leu
        275                 280                 285

Lys Gln Asn Lys Phe Ser Pro Leu Pro Leu Lys Tyr Ile Arg Pro Val
    290                 295                 300

Leu Gln Gln Val Ala Thr Ala Leu Met Lys Leu Lys Ser Leu Gly Leu
305                 310                 315                 320

Ile His Ala Asp Leu Lys Pro Glu Asn Ile Met Leu Val Asp Pro Ser
                325                 330                 335

Arg Gln Pro Tyr Arg Val Lys Val Ile Asp Phe Gly Ser Ala Ser His
            340                 345                 350

Val Ser Lys Ala Val Cys Ser Thr Tyr Leu Gln Ser Arg Tyr Tyr Arg
        355                 360                 365

Ala Pro Glu Ile Ile Leu Gly Leu Pro Phe Cys Glu Ala Ile Asp Met
    370                 375                 380

Trp Ser Leu Gly Cys Val Ile Ala Glu Leu Phe Leu Gly Trp Pro Leu
385                 390                 395                 400

Tyr Pro Gly Ala Ser Glu Tyr Asp Gln Ile Arg Tyr Ile Ser Gln Thr
                405                 410                 415

Gln Gly Leu Pro Ala Glu Tyr Leu Leu Ser Ala Gly Thr Lys Thr Thr
            420                 425                 430

Arg Phe Phe Asn Arg Asp Thr Asp Ser Pro Tyr Pro Leu Trp Arg Leu
        435                 440                 445

Lys Thr Pro Asp Asp His Glu Ala Glu Thr Gly Ile Lys Ser Lys Glu
    450                 455                 460

Ala Arg Lys Tyr Ile Phe Asn Cys Leu Asp Asp Met Ala Gln Val Asn
465                 470                 475                 480

Met Thr Thr Asp Leu Glu Gly Ser Asp Met Leu Val Glu Lys Ala Asp
                485                 490                 495

Arg Arg Glu Phe Ile Asp Leu Leu Lys Lys Met Leu Thr Ile Asp Ala
            500                 505                 510

Asp Lys Arg Ile Thr Pro Ile Glu Thr Leu Asn His Pro Phe Val Thr
        515                 520                 525

Met Thr His Leu Leu Asp Phe Pro His Ser Thr His Val Lys Ser Cys
    530                 535                 540

Phe Gln Asn Met Glu Ile Cys Lys Arg Arg Val Asn Met Tyr Asp Thr
545                 550                 555                 560

Val Asn Gln Ser Lys Thr Pro Phe Ile Thr His Val Ala Pro Ser Thr
                565                 570                 575

Ser Thr Asn Leu Thr Met Thr Phe Asn Asn Gln Leu Thr Thr Val His
            580                 585                 590

Asn Gln Ala Pro Ser Ser Thr Ser Ala Thr Ile Ser Leu Ala Asn Pro
        595                 600                 605

Glu Val Ser Ile Leu Asn Tyr Pro Ser Thr Leu Tyr Gln Pro Ser Ala
    610                 615                 620

Ala Ser Met Ala Ala Val Ala Gln Arg Ser Met Pro Leu Gln Thr Gly
625                 630                 635                 640

Thr Ala Gln Ile Cys Ala Arg Pro Asp Pro Phe Gln Gln Ala Leu Ile
                645                 650                 655

Val Cys Pro Pro Gly Phe Gln Gly Leu Gln Ala Ser Pro Ser Lys His
            660                 665                 670

Ala Gly Tyr Ser Val Arg Met Glu Asn Ala Val Pro Ile Val Thr Gln
        675                 680                 685

Ala Pro Gly Ala Gln Pro Leu Gln Ile Gln Pro Gly Leu Leu Ala Gln
    690                 695                 700

Gln Ala Trp Pro Ser Gly Thr Gln Gln Ile Leu Leu Pro Pro Ala Trp
705                 710                 715                 720

Gln Gln Leu Thr Gly Val Ala Thr His Thr Ser Val Gln His Ala Thr
                725                 730                 735

Val Ile Pro Glu Thr Met Ala Gly Thr Gln Gln Leu Ala Asp Trp Arg
            740                 745                 750

Asn Thr His Ala His Gly Ser His Tyr Asn Pro Ile Met Gln Gln Pro
        755                 760                 765

Ala Leu Leu Thr Gly His Val Thr Leu Pro Ala Ala Gln Pro Leu Asn
    770                 775                 780

Val Gly Val Ala His Val Met Arg Gln Gln Pro Thr Ser Thr Thr Ser
785                 790                 795                 800

Ser Arg Lys Ser Lys Gln His Gln Ser Ser Val Arg Asn Val Ser Thr
                805                 810                 815

Cys Glu Val Ser Ser Ser Gln Ala Ile Ser Ser Pro Gln Arg Ser Lys
            820                 825                 830

Arg Val Lys Glu Asn Thr Pro Pro Arg Cys Ala Met Val His Ser Ser
        835                 840                 845

Pro Ala Cys Ser Thr Ser Val Thr Cys Gly Trp Gly Asp Val Ala Ser
    850                 855                 860

Ser Thr Thr Arg Glu Arg Gln Arg Gln Thr Ile Val Ile Pro Asp Thr
865                 870                 875                 880

Pro Ser Pro Thr Val Ser Val Ile Thr Ile Ser Ser Asp Thr Asp Glu
                885                 890                 895

Glu Glu Glu Gln Lys His Ala Pro Thr Ser Thr Val Ser Lys Gln Arg
            900                 905                 910

Lys Asn Val Ile Ser Cys Val Thr Val His Asp Ser Pro Tyr Ser Asp
        915                 920                 925

Ser Ser Ser Asn Thr Ser Pro Tyr Ser Val Gln Gln Arg Ala Gly His
    930                 935                 940

Asn Asn Ala Asn Ala Phe Asp Thr Lys Gly Ser Leu Glu Asn His Cys
945                 950                 955                 960

Thr Gly Asn Pro Arg Thr Ile Ile Val Pro Pro Leu Lys Thr Gln Ala
                965                 970                 975

Ser Glu Val Leu Val Glu Cys Asp Ser Leu Val Pro Val
            980                 985

<210>  3

<211>  2956

<212>  DNA

<213>  Homo sapiens


<220>

<221>  CDS

<222>  (1)..(2736)

<223>

<400> 3
atg gcc tca cat gtg caa gtt ttc tcc cct cac acc ctt caa tca agt 48
gcc ttc tgt agt gtg aag aaa ctg aaa ata gag ccg agt tcc aac tgg 96
gac atg act ggg tac ggc tcc cac agc aaa gtg tat agc cag agc aag 144
aac atc ccc ctg tcg cag cca gcc acc aca acc gtc agc acc tcc ttg 192
ccg gtc cca aac cca agc cta cct tac gag cag acc atc gtc ttc cca 240
gga agc acc ggg cac atc gtg gtc acc tca gca agc agc act tct gtc 288
acc ggg caa gtc ctc ggc gga cca cac aac cta atg cgt cga agc act 336
gtg agc ctc ctt gat acc tac caa aaa tgt gga ctc aag cgt aag agc 384
gag gag atc gag aac aca agc agc gtg cag atc atc gag gag cat cca 432
ccc atg att cag aat aat gca agc ggg gcc act gtc gcc act gcc acc 480
acg ttt act gcc acc tcc aaa aac agc ggc tcc aac agc gag ggg gac 528
tat cag ctg tcg cag cat gag gtg ctg tgc tcc atg acc aac acc tac 576
gag gtc tca gag ttc ttg ggc cga ggg acg ttt ggg caa gtg gtc aag 624
tgc tgg aaa cgg ggc acc aat gag atc gta gcc atc aag atc ctg aag 672
aac cac cca tcc tat gcc cga caa ggt cag att gaa gtg agc atc ctg 720
gcc cgg ttg agc acg gag agt gcc gat gac tat aac ttc gtc cgg gcc      768
tac gaa tgc ttc cag cac aag aac cac acg tgc ttg gtc ttc gag atg      816
ttg gag cag aac ctc tat gac ttt ctg aag caa aac aag ttt agc ccc      864
ttg ccc ctc aaa tac att cgc cca gtt ctc cag cag gta gcc aca gcc      912
ctg atg aaa ctc aaa agc cta ggt ctt atc cac gct gac ctc aaa cca      960
gaa aac atc atg ctg gtg gat cca tct aga caa cca tac aga gtc aag     1008
gtc atc gac ttt ggt tca gcc agc cac gtc tcc aag gct gtg tgc tcc     1056
acc tac ttg cag tcc aga tat tac agg gcc cct gag atc atc ctt ggt     1104
tta cca ttt tgt gag gca att gac atg tgg tcc ctg ggt tgt gtt att     1152
gca gaa ttg ttc ctg ggg tgg ccg tta tat cca gga gct tcg gag tat     1200
gat cag att cgg tat att tca caa aca cag ggt ttg cct gct gaa tat     1248
tta tta agc gcc ggg aca aag aca act agg ttt ttt aac cgt gac acg     1296
gac tca cca tat cct ttg tgg aga ctg aag aca cca gat gac cat gaa     1344
gca gag aca ggg att aag tca aaa gaa gca aga aag tac att ttc aac     1392
tgt tta gat gat atg gcc cag gtg aac atg acg aca gat ttg gaa ggg     1440
agc gac atg ttg gta gaa aag gct gac cgg cgg gag ttc att gac ctg      1488
ttg aag aag atg ctg acc att gat gct gac aag aga atc act cca atc      1536
gaa acc ctg aac cat ccc ttt gtc acc atg aca cac tta ctc gat ttt      1584
ccc cac agc aca cac gtc aaa tca tgt ttc cag aac atg gag atc tgc      1632
aag cgt cgg gtg aat atg tat gac acg gtg aac cag agc aaa acc cct      1680
ttc atc acg cac gtg gcc ccc agc acg tcc acc aac ctg acc atg acc      1728
ttt aac aac cag ctg acc act gtc cac aac cag gct ccc tcc tct acc      1776
agt gcc act att tcc tta gcc aat ccc gaa gtc tcc ata cta aac tac      1824
cca tct aca ctc tac cag ccc tca gcg gca tcc atg gct gca gtg gcc      1872
cag cgg agc atg ccc ctg cag aca gga aca gcc cag att tgt gcc cgg      1920
cct gac ccg ttc cag caa gct ctc atc gtg tgt ccc ccc ggc ttc caa      1968
ggc ttg cag gcc tct tcc tct aag cac gct ggc tac tcg gtg cga atg      2016
gaa aat gca gtt ccc atc gtc act caa gcc cca gga gct cag cct ctt      2064
cag atc caa cca ggt ctg ctt gcc cag cag gct tgg cca agt ggg aac      2112
caa cag atc ctg ctt tct ccc aca tgg cag caa ctg gat gga gtg gcc      2160
acc cac aca tca gtg cag cat gcc acc gtg att ccc gag acc atg gca     2208
ggc acc cag cag ctg gcg gac tgg aga aat acg cat gct cac gga agc     2256
cat tat aat ccc atc atg cag cag cct gca cta ttg acc ggt cat gtg     2304
acc ctt cca gca gca cag ccc tta aat gtg ggt gtg gcc cac gtg atg     2352
cgg cag cag cca acc agc acc acc tcc tcc cgg aag agt aag cag cat     2400
gtc tcc aag caa aga aaa aac gtc atc agc tgt gtc aca gtc cac gac     2448
tcc ccc tac tcc gac tcc tcc agc aac acc agc ccc tac tcc gtg cag     2496
cag cgt gct ggg cac aac aat gcc aat gcc ttt gac acc aag ggg agc     2544
ctg gag aat cac tgc acg ggg aac ccc cga acc atc atc gtg cca ccc     2592
ctg aaa acc cag gcc agc gaa gta ttg gtg gag tgt gat agc ctg gtg     2640
cca ggt aat ttg ggg cca gga cag ggc agg aac ctc tcc ctg gag agt     2688
ggt ttt cct gct ttt ctg ctg cta gaa atg ttg ctg tat ggg agc tag     2736
tacaatagaa agtgcccagt gactgcaggc gacccttacc gaggatttta ttcaagtatc     2796
caggggaggc ttctgctgaa cagcacattg tataagcagc gttttccttc tttaatcctg     2856
taatcaatac ctctctgcct ttacaatggc tctgcttcca actaggttga ttaatagaaa     2916
ataaaagcct cctggttctt taaaaaaaaa aaaaaaaaaa                           2956

<210>  4

<211>  911

<212>  PRT

<213>  Homo sapiens

<400>  4

<210>  5

<211>  3507

<212>  DNA

<213>  Mus musculus

<220>

<221>  CDS

<222>  (1)..(3507)

<223>

<400>  5
atg gcc ccc gtg tac gaa ggt atg gcc tca cat gtg caa gtt ttc tcc      48
Met Ala Pro Val Tyr Glu Gly Met Ala Ser His Val Gln Val Phe Ser
1               5                   10                  15

cct cac acc ctt caa tca agt gcc ttc tgt agt gtg aag aaa cta aaa      96
Pro His Thr Leu Gln Ser Ser Ala Phe Cys Ser Val Lys Lys Leu Lys
            20                  25                  30

gta gag cca agt tcc aac tgg gac atg act ggg tac ggc tcc cac agc      144
Val Glu Pro Ser Ser Asn Trp Asp Met Thr Gly Tyr Gly Ser His Ser
        35                  40                  45

aaa gtg tac agc cag agc aag aac ata cca cct tct cag cca gcc tcc      192
Lys Val Tyr Ser Gln Ser Lys Asn Ile Pro Pro Ser Gln Pro Ala Ser
    50                  55                  60

aca acc gtc agc acc tcc ttg cca atc ccc aac cca agc cta cct tac      240
Thr Thr Val Ser Thr Ser Leu Pro Ile Pro Asn Pro Ser Leu Pro Tyr
65                  70                  75                  80

gag cag acc atc atc ttc cca gga agt act gga cac ata gtt gta aca      288
Glu Gln Thr Ile Ile Phe Pro Gly Ser Thr Gly His Ile Val Val Thr
                85                  90                  95

tca gca agt agc act tct gtc acc ggg caa gtc ctc ggt gga cca cat      336
Ser Ala Ser Ser Thr Ser Val Thr Gly Gln Val Leu Gly Gly Pro His
            100                 105                 110

aac cta atg cgt cga agc act gtg agc ctc ctt gac acc tac caa aaa      384
Asn Leu Met Arg Arg Ser Thr Val Ser Leu Leu Asp Thr Tyr Gln Lys
        115                 120                 125

tgt gga ctc aag cgc aag agt gag gag att gag aac aca agc agc gtg      432
Cys Gly Leu Lys Arg Lys Ser Glu Glu Ile Glu Asn Thr Ser Ser Val
    130                 135                 140

cag atc att gaa gag cat cca ccc atg att cag aat aat gcc agc ggg      480
Gln Ile Ile Glu Glu His Pro Pro Met Ile Gln Asn Asn Ala Ser Gly
145                 150                 155                 160

gcc act gta gcc act gcc acc aca tcc act gcc acc tcc aaa aac agt      528
Ala Thr Val Ala Thr Ala Thr Thr Ser Thr Ala Thr Ser Lys Asn Ser
                165                 170                 175

ggc tcc aac agt gaa ggg gac tat cag ctg gtg cag cat gaa gta ctg      576
Gly Ser Asn Ser Glu Gly Asp Tyr Gln Leu Val Gln His Glu Val Leu
            180                 185                 190

tgc tcc atg acc aac acc tat gag gtt tta gag ttc ttg ggg cga ggg 624
Cys Ser Met Thr Asn Thr Tyr Glu Val Leu Glu Phe Leu Gly Arg Gly
        195                 200                 205

aca ttt ggg caa gtg gtc aag tgc tgg aaa cgg ggc acc aat gaa att 672
Thr Phe Gly Gln Val Val Lys Cys Trp Lys Arg Gly Thr Asn Glu Ile
    210                 215                 220

gtg gcc att aag atc ctc aag aac cac ccc tcc tat gcc cgg cag ggc 720
Val Ala Ile Lys Ile Leu Lys Asn His Pro Ser Tyr Ala Arg Gln Gly
225                 230                 235                 240

cag att gaa gtg agc atc ttg gcc cgg ctg agc acg gag agc gct gat 768
Gln Ile Glu Val Ser Ile Leu Ala Arg Leu Ser Thr Glu Ser Ala Asp
                245                 250                 255

gac tac aat ttt gtg cgg gcc tat gag tgc ttc cag cac aag aac cac 816
Asp Tyr Asn Phe Val Arg Ala Tyr Glu Cys Phe Gln His Lys Asn His
            260                 265                 270

acg tgc tta gtc ttt gag atg ttg gag cag aac ctt tat gac ttt ctg 864
Thr Cys Leu Val Phe Glu Met Leu Glu Gln Asn Leu Tyr Asp Phe Leu
        275                 280                 285

aaa cag aac aag ttt agt ccc ttg ccc ctc aag tat att cgc cca gtc 912
Lys Gln Asn Lys Phe Ser Pro Leu Pro Leu Lys Tyr Ile Arg Pro Val
    290                 295                 300

ctc cag cag gta gcc aca gcc ctg atg aaa ctc aaa agc ctg ggt ctt 960
Leu Gln Gln Val Ala Thr Ala Leu Met Lys Leu Lys Ser Leu Gly Leu
305                 310                 315                 320

atc cat gct gac ctc aaa cca gaa aac atc atg ctg gtg gat cca tcc 1008
Ile His Ala Asp Leu Lys Pro Glu Asn Ile Met Leu Val Asp Pro Ser
                325                 330                 335

aga caa ccg tac cga gtg aag gtc att gac ttt ggt tca gct agt cat 1056
Arg Gln Pro Tyr Arg Val Lys Val Ile Asp Phe Gly Ser Ala Ser His
            340                 345                 350

gtg tcc aaa gct gtc tgc tct acg tac ttg caa tcc aga tac tac cgg 1104
Val Ser Lys Ala Val Cys Ser Thr Tyr Leu Gln Ser Arg Tyr Tyr Arg
        355                 360                 365

gcc cct gag atc atc ctt ggt ttg cca ttc tgt gag gca att gac atg 1152
Ala Pro Glu Ile Ile Leu Gly Leu Pro Phe Cys Glu Ala Ile Asp Met
    370                 375                 380

tgg tcc ctt ggt tgt gtc att gct gaa ctg ttc ctg ggc tgg cca tta 1200
Trp Ser Leu Gly Cys Val Ile Ala Glu Leu Phe Leu Gly Trp Pro Leu
385                 390                 395                 400

tac cca ggt gct tct gag tat gat cag att cgg tat att tca caa aca 1248
Tyr Pro Gly Ala Ser Glu Tyr Asp Gln Ile Arg Tyr Ile Ser Gln Thr
                405                 410                 415

cag ggt tta cca gct gaa tat tta tta agt gca ggg aca aag aca act 1296
Gln Gly Leu Pro Ala Glu Tyr Leu Leu Ser Ala Gly Thr Lys Thr Thr
            420                 425                 430

agg ttt ttc aac cgt gac aca gac tca ccg tat cct ttg tgg agg cta 1344
Arg Phe Phe Asn Arg Asp Thr Asp Ser Pro Tyr Pro Leu Trp Arg Leu
        435                 440                 445

aag aca cca gat gac cat gaa gca gag acg gga att aag tca aag gaa 1392
Lys Thr Pro Asp Asp His Glu Ala Glu Thr Gly Ile Lys Ser Lys Glu
    450                 455                 460

gca aga aag tac att ttc aac tgt ttg gat gat atg gcc cag gtg aac 1440
Ala Arg Lys Tyr Ile Phe Asn Cys Leu Asp Asp Met Ala Gln Val Asn
465                 470                 475                 480

atg aca aca gat ttg gaa ggg agt gac atg tta gta gag aag gca gac 1488
Met Thr Thr Asp Leu Glu Gly Ser Asp Met Leu Val Glu Lys Ala Asp
                485                 490                 495

cga cgg gag ttc att gac ctg tta aag aag atg ctg acc atc gat gct 1536
Arg Arg Glu Phe Ile Asp Leu Leu Lys Lys Met Leu Thr Ile Asp Ala
            500                 505                 510

gat aag aga gtc act ccc att gaa act ctg aac cac ccc ttt gtc acc 1584
Asp Lys Arg Val Thr Pro Ile Glu Thr Leu Asn His Pro Phe Val Thr
        515                 520                 525

atg aca cac ctg ctt gac ttc ccc cat agt gcc cat gtc aaa tct tgt 1632
Met Thr His Leu Leu Asp Phe Pro His Ser Ala His Val Lys Ser Cys
    530                 535                 540

ttc caa aac atg gag att tgc aag cgc cgg gtg aat atg tat gac aca 1680
Phe Gln Asn Met Glu Ile Cys Lys Arg Arg Val Asn Met Tyr Asp Thr
545                 550                 555                 560

gtg aac cag agc aaa aca cct ttc atc act cac gtg gcc ccc agc aca 1728
Val Asn Gln Ser Lys Thr Pro Phe Ile Thr His Val Ala Pro Ser Thr
                565                 570                 575

tcc acc aac ttg acc atg acc ttt aac aac cag ctg acc act gtc cac 1776
Ser Thr Asn Leu Thr Met Thr Phe Asn Asn Gln Leu Thr Thr Val His
            580                 585                 590

aac cag ccc tca gcg gca tcc atg gcc gcg gtg gcc ccg cgg agc atg 1824
Asn Gln Pro Ser Ala Ala Ser Met Ala Ala Val Ala Pro Arg Ser Met
        595                 600                 605

ccc ctg cag acg gga aca gcc caa att tgt gcc cga cct gat ccc ttc 1872
Pro Leu Gln Thr Gly Thr Ala Gln Ile Cys Ala Arg Pro Asp Pro Phe
    610                 615                 620

cag caa gct ctc atc gtg tgt ccc cct ggc ttc caa ggc ctg cag gcc 1920
Gln Gln Ala Leu Ile Val Cys Pro Pro Gly Phe Gln Gly Leu Gln Ala
625                 630                 635                 640

tct ccc tcc aag cac gct ggc tac tca gtg cgg atg gaa aat gct gtc 1968
Ser Pro Ser Lys His Ala Gly Tyr Ser Val Arg Met Glu Asn Ala Val
                645                 650                 655

ccc atc gtc acc cag gcg cca gga gct cag cct ctt cag atc caa cca 2016
Pro Ile Val Thr Gln Ala Pro Gly Ala Gln Pro Leu Gln Ile Gln Pro
            660                 665                 670

ggc ctg ctt gct cag gcc tgg cca ggt ggg gcc caa cag atc cta ctt 2064

Gly Leu Leu Ala Gln Ala Trp Pro Gly Gly Ala Gln Gln Ile Leu Leu
        675                 680                 685

cct cca gca tgg cag cag ctg act ggc gtg gcc acc cac aca tct gta 2112
Pro Pro Ala Trp Gln Gln Leu Thr Gly Val Ala Thr His Thr Ser Val
    690                 695                 700

cag cat gca gct gtg att cct gag acc atg gca ggc act cag caa ctg 2160
Gln His Ala Ala Val Ile Pro Glu Thr Met Ala Gly Thr Gln Gln Leu
705                 710                 715                 720

gct gac tgg agg aac acg cat gct cat gga agt cat tac aat ccc atc 2208
Ala Asp Trp Arg Asn Thr His Ala His Gly Ser His Tyr Asn Pro Ile
                725                 730                 735

atg cag cag cct gca ctc ttg act ggt cat gtg acc ctt cca gct gcc 2256
Met Gln Gln Pro Ala Leu Leu Thr Gly His Val Thr Leu Pro Ala Ala
            740                 745                 750

cag ccc tta aat gtg ggt gta gcc cat gtg atg agg caa caa cca acc 2304
Gln Pro Leu Asn Val Gly Val Ala His Val Met Arg Gln Gln Pro Thr
        755                 760                 765

agc acc acc tcc tcc agg aag agt aag cag cac cag tca tct gtg aga 2352
Ser Thr Thr Ser Ser Arg Lys Ser Lys Gln His Gln Ser Ser Val Arg
    770                 775                 780

aat gtc tcc acc tgt gag gtg acc tct tca cag gct atc agc tcc cct 2400
Asn Val Ser Thr Cys Glu Val Thr Ser Ser Gln Ala Ile Ser Ser Pro
785                 790                 795                 800

cag cga tcc aag cgt gtc aag gag aac act ccc cca cgg tgc gcc atg 2448
Gln Arg Ser Lys Arg Val Lys Glu Asn Thr Pro Pro Arg Cys Ala Met
                805                 810                 815

gta cac agc agc cca gct tgc agc acc tca gtc acc tgt ggg tgg ggc 2496
Val His Ser Ser Pro Ala Cys Ser Thr Ser Val Thr Cys Gly Trp Gly
            820                 825                 830

gac gtg gcc tcc agc acc acc cgg gag cga cag cgg cag acg ata gtc 2544
Asp Val Ala Ser Ser Thr Thr Arg Glu Arg Gln Arg Gln Thr Ile Val
        835                 840                 845

atc ccc gac acc ccg agc ccc aca gtc agt gtc atc acc atc agc agt 2592
Ile Pro Asp Thr Pro Ser Pro Thr Val Ser Val Ile Thr Ile Ser Ser
    850                 855                 860

gac acc gat gaa gaa gag gag cag aag cac gcc ccc acc agc aca gtc 2640
Asp Thr Asp Glu Glu Glu Glu Gln Lys His Ala Pro Thr Ser Thr Val
865                 870                 875                 880

tcc aag caa aga aaa aat gtc atc agc tgt gtc acc gtc cac gac tct 2688
Ser Lys Gln Arg Lys Asn Val Ile Ser Cys Val Thr Val His Asp Ser
                885                 890                 895

ccc tac tct gac tcc tcc agc aac acc agc ccc tac tcg gtg cag cag 2736
Pro Tyr Ser Asp Ser Ser Ser Asn Thr Ser Pro Tyr Ser Val Gln Gln
            900                 905                 910

cgc aca ggg cac aac ggc acc aac acc ttg gac acc aag ggg ggc cta 2784
Arg Thr Gly His Asn Gly Thr Asn Thr Leu Asp Thr Lys Gly Gly Leu

915                 920                 925

gag aat cac tgc act ggc aac ccc cgc acc atc atc gta ccc cca ctg     2832
Glu Asn His Cys Thr Gly Asn Pro Arg Thr Ile Ile Val Pro Pro Leu
    930                 935                 940

aag acc cag gcc agt gaa gtg ttg gta gaa tgt gac agc cta ggg cca     2880
Lys Thr Gln Ala Ser Glu Val Leu Val Glu Cys Asp Ser Leu Gly Pro
945                 950                 955                 960

gcg atc agt gcc agt cac cat tcg tcc tcc ttc aag tcc aag tcc tcc     2928
Ala Ile Ser Ala Ser His His Ser Ser Ser Phe Lys Ser Lys Ser Ser
                965                 970                 975

agc acc gtg acc tcc acc agt gga cac tct tca ggg agc tct tca gga     2976
Ser Thr Val Thr Ser Thr Ser Gly His Ser Ser Gly Ser Ser Ser Gly
            980                 985                 990

gcc atc gcc tac cgt cag cag cgg  cca ggc ccc cac ttc  cag cag cag     3024
Ala Ile Ala Tyr Arg Gln Gln Arg  Pro Gly Pro His Phe  Gln Gln Gln
        995                 1000                 1005

cag ccc  ctc aat ctc agc cag  gcc cag cag cac atg  gct gcg gac     3069
Gln Pro  Leu Asn Leu Ser Gln  Ala Gln Gln His Met  Ala Ala Asp
    1010                 1015                 1020

cgc acc  ggg agt cac cgt cga  cag cag gcc tac atc  act ccg acc     3114
Arg Thr  Gly Ser His Arg Arg  Gln Gln Ala Tyr Ile  Thr Pro Thr
    1025                 1030                 1035

atg gcg  caa gct ccg tac acc  ttc ccg cac aac agc  ccc agc cat     3159
Met Ala  Gln Ala Pro Tyr Thr  Phe Pro His Asn Ser  Pro Ser His
    1040                 1045                 1050

ggc act  gtt cac ccc cac ctg  gct gcg gct gcc cac  ctc ccc acc     3204
Gly Thr  Val His Pro His Leu  Ala Ala Ala Ala His  Leu Pro Thr
    1055                 1060                 1065

cag cct  cac ctc tac acc tat  aca gcg ccc aca gcc  cta ggc tcc     3249
Gln Pro  His Leu Tyr Thr Tyr  Thr Ala Pro Thr Ala  Leu Gly Ser
    1070                 1075                 1080

acc ggc  act gta gcc cac ctg  gtg gca tcc caa ggc  tca gca cgc     3294
Thr Gly  Thr Val Ala His Leu  Val Ala Ser Gln Gly  Ser Ala Arg
    1085                 1090                 1095

cac acc  gtg cag cac act gcc  tat ccg gcc agc atc  gtc cac cag     3339
His Thr  Val Gln His Thr Ala  Tyr Pro Ala Ser Ile  Val His Gln
    1100                 1105                 1110

gtc cct  gtg agc atg ggg ccc  cgg gtc ctg ccc tcg  ccc acc atc     3384
Val Pro  Val Ser Met Gly Pro  Arg Val Leu Pro Ser  Pro Thr Ile
    1115                 1120                 1125

cac ccc  agt cag tat cca gcc  cag ttt gcc cac cag  acc tat atc     3429
His Pro  Ser Gln Tyr Pro Ala  Gln Phe Ala His Gln  Thr Tyr Ile
    1130                 1135                 1140

agc gcc  tcg cca gcc tcc acc  gtc tac act gga tac  cca ctg agt     3474
Ser Ala  Ser Pro Ala Ser Thr  Val Tyr Thr Gly Tyr  Pro Leu Ser
    1145                 1150                 1155

cct gcc aag gtc aac caa tac cct tac ata taa 3507
Pro Ala Lys Val Asn Gln Tyr Pro Tyr Ile
    1160                1165

<210> 6

<211> 1168

<212> PRT

<213> Mus musculus

<400> 6

Met Ala Pro Val Tyr Glu Gly Met Ala Ser His Val Gln Val Phe Ser
1               5                   10                  15

Pro His Thr Leu Gln Ser Ser Ala Phe Cys Ser Val Lys Lys Leu Lys
            20                  25                  30

Val Glu Pro Ser Ser Asn Trp Asp Met Thr Gly Tyr Gly Ser His Ser
        35                  40                  45

Lys Val Tyr Ser Gln Ser Lys Asn Ile Pro Pro Ser Gln Pro Ala Ser
    50                  55                  60

Thr Thr Val Ser Thr Ser Leu Pro Ile Pro Asn Pro Ser Leu Pro Tyr
65                  70                  75                  80

Glu Gln Thr Ile Ile Phe Pro Gly Ser Thr Gly His Ile Val Val Thr
                85                  90                  95

Ser Ala Ser Ser Thr Ser Val Thr Gly Gln Val Leu Gly Gly Pro His
            100                 105                 110

Asn Leu Met Arg Arg Ser Thr Val Ser Leu Leu Asp Thr Tyr Gln Lys
        115                 120                 125

Cys Gly Leu Lys Arg Lys Ser Glu Glu Ile Glu Asn Thr Ser Ser Val
    130                 135                 140

Gln Ile Ile Glu Glu His Pro Pro Met Ile Gln Asn Asn Ala Ser Gly
145                 150                 155                 160

Ala Thr Val Ala Thr Ala Thr Thr Ser Thr Ala Thr Ser Lys Asn Ser
                165                 170                 175

Gly Ser Asn Ser Glu Gly Asp Tyr Gln Leu Val Gln His Glu Val Leu
            180                 185                 190

Cys Ser Met Thr Asn Thr Tyr Glu Val Leu Glu Phe Leu Gly Arg Gly
        195                 200                 205

Thr Phe Gly Gln Val Val Lys Cys Trp Lys Arg Gly Thr Asn Glu Ile
    210                 215                 220

Val Ala Ile Lys Ile Leu Lys Asn His Pro Ser Tyr Ala Arg Gln Gly
225                 230                 235                 240

Gln Ile Glu Val Ser Ile Leu Ala Arg Leu Ser Thr Glu Ser Ala Asp
                245                 250                 255

Asp Tyr Asn Phe Val Arg Ala Tyr Glu Cys Phe Gln His Lys Asn His
            260                 265                 270

Thr Cys Leu Val Phe Glu Met Leu Glu Gln Asn Leu Tyr Asp Phe Leu
        275                 280                 285

Lys Gln Asn Lys Phe Ser Pro Leu Pro Leu Lys Tyr Ile Arg Pro Val
    290                 295                 300

Leu Gln Gln Val Ala Thr Ala Leu Met Lys Leu Lys Ser Leu Gly Leu
305                 310                 315                 320

Ile His Ala Asp Leu Lys Pro Glu Asn Ile Met Leu Val Asp Pro Ser
                325                 330                 335

Arg Gln Pro Tyr Arg Val Lys Val Ile Asp Phe Gly Ser Ala Ser His
            340                 345                 350

Val Ser Lys Ala Val Cys Ser Thr Tyr Leu Gln Ser Arg Tyr Tyr Arg
        355                 360                 365

Ala Pro Glu Ile Ile Leu Gly Leu Pro Phe Cys Glu Ala Ile Asp Met
    370                 375                 380

Trp Ser Leu Gly Cys Val Ile Ala Glu Leu Phe Leu Gly Trp Pro Leu
385                 390                 395                 400

Tyr Pro Gly Ala Ser Glu Tyr Asp Gln Ile Arg Tyr Ile Ser Gln Thr
                405                 410                 415

Gln Gly Leu Pro Ala Glu Tyr Leu Leu Ser Ala Gly Thr Lys Thr Thr
            420                 425                 430

Arg Phe Phe Asn Arg Asp Thr Asp Ser Pro Tyr Pro Leu Trp Arg Leu
        435                 440                 445

Lys Thr Pro Asp Asp His Glu Ala Glu Thr Gly Ile Lys Ser Lys Glu
    450                 455                 460

Ala Arg Lys Tyr Ile Phe Asn Cys Leu Asp Asp Met Ala Gln Val Asn
465                 470                 475                 480

Met Thr Thr Asp Leu Glu Gly Ser Asp Met Leu Val Glu Lys Ala Asp
                485                 490                 495

Arg Arg Glu Phe Ile Asp Leu Leu Lys Lys Met Leu Thr Ile Asp Ala
            500                 505                 510

Asp Lys Arg Val Thr Pro Ile Glu Thr Leu Asn His Pro Phe Val Thr
        515                 520                 525

Met Thr His Leu Leu Asp Phe Pro His Ser Ala His Val Lys Ser Cys
    530                 535                 540

Phe Gln Asn Met Glu Ile Cys Lys Arg Arg Val Asn Met Tyr Asp Thr
545                 550                 555                 560

Val Asn Gln Ser Lys Thr Pro Phe Ile Thr His Val Ala Pro Ser Thr
                565                 570                 575

Ser Thr Asn Leu Thr Met Thr Phe Asn Asn Gln Leu Thr Thr Val His
            580                 585                 590

Asn Gln Pro Ser Ala Ala Ser Met Ala Ala Val Ala Pro Arg Ser Met
        595                 600                 605

Pro Leu Gln Thr Gly Thr Ala Gln Ile Cys Ala Arg Pro Asp Pro Phe
    610                 615                 620

Gln Gln Ala Leu Ile Val Cys Pro Pro Gly Phe Gln Gly Leu Gln Ala
625                 630                 635                 640

Ser Pro Ser Lys His Ala Gly Tyr Ser Val Arg Met Glu Asn Ala Val
                645                 650                 655

Pro Ile Val Thr Gln Ala Pro Gly Ala Gln Pro Leu Gln Ile Gln Pro

660                 665                 670

Gly Leu Leu Ala Gln Ala Trp Pro Gly Gly Ala Gln Gln Ile Leu Leu
        675                 680                 685

Pro Pro Ala Trp Gln Gln Leu Thr Gly Val Ala Thr His Thr Ser Val
    690                 695                 700

Gln His Ala Ala Val Ile Pro Glu Thr Met Ala Gly Thr Gln Gln Leu
705                 710                 715                 720

Ala Asp Trp Arg Asn Thr His Ala His Gly Ser His Tyr Asn Pro Ile
                725                 730                 735

Met Gln Gln Pro Ala Leu Leu Thr Gly His Val Thr Leu Pro Ala Ala
            740                 745                 750

Gln Pro Leu Asn Val Gly Val Ala His Val Met Arg Gln Gln Pro Thr
        755                 760                 765

Ser Thr Thr Ser Ser Arg Lys Ser Lys Gln His Gln Ser Ser Val Arg
    770                 775                 780

Asn Val Ser Thr Cys Glu Val Thr Ser Ser Gln Ala Ile Ser Ser Pro
785                 790                 795                 800

Gln Arg Ser Lys Arg Val Lys Glu Asn Thr Pro Pro Arg Cys Ala Met
                805                 810                 815

Val His Ser Ser Pro Ala Cys Ser Thr Ser Val Thr Cys Gly Trp Gly
            820                 825                 830

Asp Val Ala Ser Ser Thr Thr Arg Glu Arg Gln Arg Gln Thr Ile Val
        835                 840                 845

Ile Pro Asp Thr Pro Ser Pro Thr Val Ser Val Ile Thr Ile Ser Ser
    850                 855                 860

Asp Thr Asp Glu Glu Glu Glu Gln Lys His Ala Pro Thr Ser Thr Val
865                 870                 875                 880

Ser Lys Gln Arg Lys Asn Val Ile Ser Cys Val Thr Val His Asp Ser
                885                 890                 895

Pro Tyr Ser Asp Ser Ser Ser Asn Thr Ser Pro Tyr Ser Val Gln Gln
            900                 905                 910

Arg Thr Gly His Asn Gly Thr Asn Thr Leu Asp Thr Lys Gly Gly Leu
        915                 920                 925

Glu Asn His Cys Thr Gly Asn Pro Arg Thr Ile Ile Val Pro Pro Leu
    930                 935                 940

Lys Thr Gln Ala Ser Glu Val Leu Val Glu Cys Asp Ser Leu Gly Pro
945                 950                 955                 960

Ala Ile Ser Ala Ser His His Ser Ser Ser Phe Lys Ser Lys Ser Ser
                965                 970                 975

Ser Thr Val Thr Ser Thr Ser Gly His Ser Ser Gly Ser Ser Ser Gly
            980                 985                 990

Ala Ile Ala Tyr Arg Gln Gln Arg Pro Gly Pro His Phe Gln Gln Gln
        995                 1000                1005

Gln Pro Leu Asn Leu Ser Gln Ala Gln Gln His Met Ala Ala Asp
    1010                1015                1020

Arg Thr Gly Ser His Arg Arg Gln Gln Ala Tyr Ile Thr Pro Thr
    1025                1030                1035

Met Ala Gln Ala Pro Tyr Thr Phe Pro His Asn Ser Pro Ser His
    1040                1045                1050

Gly Thr Val His Pro His Leu Ala Ala Ala Ala His Leu Pro Thr
    1055                1060                1065

Gln Pro His Leu Tyr Thr Tyr Thr Ala Pro Thr Ala Leu Gly Ser
    1070                1075                1080

Thr Gly Thr Val Ala His Leu Val Ala Ser Gln Gly Ser Ala Arg
    1085                1090                1095

His Thr Val Gln His Thr Ala Tyr Pro Ala Ser Ile Val His Gln
    1100                1105                1110

Val Pro Val Ser Met Gly Pro Arg Val Leu Pro Ser Pro Thr Ile
    1115                1120                1125

His Pro Ser Gln Tyr Pro Ala Gln Phe Ala His Gln Thr Tyr Ile
    1130                1135                1140

Ser Ala Ser Pro Ala Ser Thr Val Tyr Thr Gly Tyr Pro Leu Ser
    1145                1150                1155

Pro Ala Lys Val Asn Gln Tyr Pro Tyr Ile
    1160                1165

<210> 7

<211> 3591

<212> DNA

<213> Mus musculus

<220>

<221> CDS

<222> (1)..(3591)

<223>

<400> 7
atg gcc ccc gtg tac gaa ggt atg gcc tca cat gtg caa gtt ttc tcc      48
Met Ala Pro Val Tyr Glu Gly Met Ala Ser His Val Gln Val Phe Ser
1               5                   10                  15

cct cac acc ctt caa tca agt gcc ttc tgt agt gtg aag aaa cta aaa      96
Pro His Thr Leu Gln Ser Ser Ala Phe Cys Ser Val Lys Lys Leu Lys
            20                  25                  30

gta gag cca agt tcc aac tgg gac atg act ggg tac ggc tcc cac agc      144
Val Glu Pro Ser Ser Asn Trp Asp Met Thr Gly Tyr Gly Ser His Ser
        35                  40                  45

aaa gtg tac agc cag agc aag aac ata cca cct tct cag cca gcc tcc      192
Lys Val Tyr Ser Gln Ser Lys Asn Ile Pro Pro Ser Gln Pro Ala Ser
    50                  55                  60

aca acc gtc agc acc tcc ttg cca atc ccc aac cca agc cta cct tac      240
Thr Thr Val Ser Thr Ser Leu Pro Ile Pro Asn Pro Ser Leu Pro Tyr
65                  70                  75                  80

gag cag acc atc atc ttc cca gga agt act gga cac ata gtt gta aca      288
Glu Gln Thr Ile Ile Phe Pro Gly Ser Thr Gly His Ile Val Val Thr
                85                  90                  95

tca gca agt agc act tct gtc acc ggg caa gtc ctc ggt gga cca cat      336
Ser Ala Ser Ser Thr Ser Val Thr Gly Gln Val Leu Gly Gly Pro His
            100                 105                 110

aac cta atg cgt cga agc act gtg agc ctc ctt gac acc tac caa aaa      384
Asn Leu Met Arg Arg Ser Thr Val Ser Leu Leu Asp Thr Tyr Gln Lys
        115                 120                 125

tgt gga ctc aag cgc aag agt gag gag att gag aac aca agc agc gtg     432
Cys Gly Leu Lys Arg Lys Ser Glu Glu Ile Glu Asn Thr Ser Ser Val
    130                 135                 140

cag atc att gaa gag cat cca ccc atg att cag aat aat gcc agc ggg     480
Gln Ile Ile Glu Glu His Pro Pro Met Ile Gln Asn Asn Ala Ser Gly
145                 150                 155                 160

gcc act gta gcc act gcc acc aca tcc act gcc acc tcc aaa aac agt     528
Ala Thr Val Ala Thr Ala Thr Thr Ser Thr Ala Thr Ser Lys Asn Ser
                165                 170                 175

ggc tcc aac agt gaa ggg gac tat cag ctg gtg cag cat gaa gta ctg     576
Gly Ser Asn Ser Glu Gly Asp Tyr Gln Leu Val Gln His Glu Val Leu
            180                 185                 190

tgc tcc atg acc aac acc tat gag gtt tta gag ttc ttg ggg cga ggg     624
Cys Ser Met Thr Asn Thr Tyr Glu Val Leu Glu Phe Leu Gly Arg Gly
        195                 200                 205

aca ttt ggg caa gtg gtc aag tgc tgg aaa cgg ggc acc aat gaa att     672
Thr Phe Gly Gln Val Val Lys Cys Trp Lys Arg Gly Thr Asn Glu Ile
    210                 215                 220

gtg gcc att aag atc ctc aag aac cac ccc tcc tat gcc cgg cag ggc     720
Val Ala Ile Lys Ile Leu Lys Asn His Pro Ser Tyr Ala Arg Gln Gly
225                 230                 235                 240

cag att gaa gtg agc atc ttg gcc cgg ctg agc acg gag agc gct gat     768
Gln Ile Glu Val Ser Ile Leu Ala Arg Leu Ser Thr Glu Ser Ala Asp
                245                 250                 255

gac tac aat ttt gtg cgg gcc tat gag tgc ttc cag cac aag aac cac     816
Asp Tyr Asn Phe Val Arg Ala Tyr Glu Cys Phe Gln His Lys Asn His
            260                 265                 270

acg tgc tta gtc ttt gag atg ttg gag cag aac ctt tat gac ttt ctg     864
Thr Cys Leu Val Phe Glu Met Leu Glu Gln Asn Leu Tyr Asp Phe Leu
        275                 280                 285

aaa cag aac aag ttt agt ccc ttg ccc ctc aag tat att cgc cca gtc     912
Lys Gln Asn Lys Phe Ser Pro Leu Pro Leu Lys Tyr Ile Arg Pro Val
    290                 295                 300

ctc cag cag gta gcc aca gcc ctg atg aaa ctc aaa agc ctg ggt ctt     960
Leu Gln Gln Val Ala Thr Ala Leu Met Lys Leu Lys Ser Leu Gly Leu
305                 310                 315                 320

atc cat gct gac ctc aaa cca gaa aac atc atg ctg gtg gat cca tcc     1008
Ile His Ala Asp Leu Lys Pro Glu Asn Ile Met Leu Val Asp Pro Ser
                325                 330                 335

aga caa ccg tac cga gtg aag gtc att gac ttt ggt tca gct agt cat     1056
Arg Gln Pro Tyr Arg Val Lys Val Ile Asp Phe Gly Ser Ala Ser His
            340                 345                 350

gtg tcc aaa gct gtc tgc tct acg tac ttg caa tcc aga tac tac cgg     1104
Val Ser Lys Ala Val Cys Ser Thr Tyr Leu Gln Ser Arg Tyr Tyr Arg
        355                 360                 365

gcc cct gag atc atc ctt ggt ttg cca ttc tgt gag gca att gac atg     1152
Ala Pro Glu Ile Ile Leu Gly Leu Pro Phe Cys Glu Ala Ile Asp Met
    370                 375                 380

tgg tcc ctt ggt tgt gtc att gct gaa ctg ttc ctg ggc tgg cca tta     1200
Trp Ser Leu Gly Cys Val Ile Ala Glu Leu Phe Leu Gly Trp Pro Leu
385                 390                 395                 400

tac cca ggt gct tct gag tat gat cag att cgg tat att tca caa aca     1248
Tyr Pro Gly Ala Ser Glu Tyr Asp Gln Ile Arg Tyr Ile Ser Gln Thr
                405                 410                 415

cag ggt tta cca gct gaa tat tta tta agt gca ggg aca aag aca act     1296
Gln Gly Leu Pro Ala Glu Tyr Leu Leu Ser Ala Gly Thr Lys Thr Thr
            420                 425                 430

agg ttt ttc aac cgt gac aca gac tca ccg tat cct ttg tgg agg cta     1344
Arg Phe Phe Asn Arg Asp Thr Asp Ser Pro Tyr Pro Leu Trp Arg Leu
        435                 440                 445

aag aca cca gat gac cat gaa gca gag acg gga att aag tca aag gaa     1392
Lys Thr Pro Asp Asp His Glu Ala Glu Thr Gly Ile Lys Ser Lys Glu
    450                 455                 460

gca aga aag tac att ttc aac tgt ttg gat gat atg gcc cag gtg aac     1440
Ala Arg Lys Tyr Ile Phe Asn Cys Leu Asp Asp Met Ala Gln Val Asn
465                 470                 475                 480

atg aca aca gat ttg gaa ggg agt gac atg tta gta gag aag gca gac     1488
Met Thr Thr Asp Leu Glu Gly Ser Asp Met Leu Val Glu Lys Ala Asp
                485                 490                 495

cga cgg gag ttc att gac ctg tta aag aag atg ctg acc atc gat gct     1536
Arg Arg Glu Phe Ile Asp Leu Leu Lys Lys Met Leu Thr Ile Asp Ala
            500                 505                 510

gat aag aga gtc act ccc att gaa act ctg aac cac ccc ttt gtc acc     1584
Asp Lys Arg Val Thr Pro Ile Glu Thr Leu Asn His Pro Phe Val Thr
        515                 520                 525

atg aca cac ctg ctt gac ttc ccc cat agt gcc cat gtc aaa tct tgt     1632
Met Thr His Leu Leu Asp Phe Pro His Ser Ala His Val Lys Ser Cys
    530                 535                 540

ttc caa aac atg gag att tgc aag cgc cgg gtg aat atg tat gac aca     1680
Phe Gln Asn Met Glu Ile Cys Lys Arg Arg Val Asn Met Tyr Asp Thr
545                 550                 555                 560

gtg aac cag agc aaa aca cct ttc atc act cac gtg gcc ccc agc aca     1728
Val Asn Gln Ser Lys Thr Pro Phe Ile Thr His Val Ala Pro Ser Thr
                565                 570                 575

tcc acc aac ttg acc atg acc ttt aac aac cag ctg acc act gtc cac     1776
Ser Thr Asn Leu Thr Met Thr Phe Asn Asn Gln Leu Thr Thr Val His
            580                 585                 590

aac cag gct ccc acc acc tcc agt gcc act ctt tcc tta gcc aat ccc     1824
Asn Gln Ala Pro Thr Thr Ser Ser Ala Thr Leu Ser Leu Ala Asn Pro
        595                 600                 605

gaa gtc tcc ata cta aac tac caa tct gca ctc tac cag ccc tca gcg     1872

Glu Val Ser Ile Leu Asn Tyr Gln Ser Ala Leu Tyr Gln Pro Ser Ala
    610                 615                 620

gca tcc atg gcc gcg gtg gcc ccg cgg agc atg ccc ctg cag acg gga      1920
Ala Ser Met Ala Ala Val Ala Pro Arg Ser Met Pro Leu Gln Thr Gly
625                 630                 635                 640

aca gcc caa att tgt gcc cga cct gat ccc ttc cag caa gct ctc atc      1968
Thr Ala Gln Ile Cys Ala Arg Pro Asp Pro Phe Gln Gln Ala Leu Ile
                645                 650                 655

gtg tgt ccc cct ggc ttc caa ggc ctg cag gcc tct ccc tcc aag cac      2016
Val Cys Pro Pro Gly Phe Gln Gly Leu Gln Ala Ser Pro Ser Lys His
            660                 665                 670

gct ggc tac tca gtg cgg atg gaa aat gct gtc ccc atc gtc acc cag      2064
Ala Gly Tyr Ser Val Arg Met Glu Asn Ala Val Pro Ile Val Thr Gln
        675                 680                 685

gcg cca gga gct cag cct ctt cag atc caa cca ggc ctg ctt gct cag      2112
Ala Pro Gly Ala Gln Pro Leu Gln Ile Gln Pro Gly Leu Leu Ala Gln
    690                 695                 700

cag gcc tgg cca ggt ggg gcc caa cag atc cta ctt cct cca gca tgg      2160
Gln Ala Trp Pro Gly Gly Ala Gln Gln Ile Leu Leu Pro Pro Ala Trp
705                 710                 715                 720

cag cag ctg act ggc gtg gcc acc cac aca tct gta cag cat gca gct      2208
Gln Gln Leu Thr Gly Val Ala Thr His Thr Ser Val Gln His Ala Ala
                725                 730                 735

gtg att cct gag acc atg gca ggc act cag caa ctg gct gac tgg agg      2256
Val Ile Pro Glu Thr Met Ala Gly Thr Gln Gln Leu Ala Asp Trp Arg
            740                 745                 750

aac acg cat gct cat gga agt cat tac aat ccc atc atg cag cag cct      2304
Asn Thr His Ala His Gly Ser His Tyr Asn Pro Ile Met Gln Gln Pro
        755                 760                 765

gca ctc ttg act ggt cat gtg acc ctt cca gct gcc cag ccc tta aat      2352
Ala Leu Leu Thr Gly His Val Thr Leu Pro Ala Ala Gln Pro Leu Asn
    770                 775                 780

gtg ggt gta gcc cat gtg atg agg caa caa cca acc agc acc acc tcc      2400
Val Gly Val Ala His Val Met Arg Gln Gln Pro Thr Ser Thr Thr Ser
785                 790                 795                 800

tcc agg aag agt aag cag cac cag tca tct gtg aga aat gtc tcc acc      2448
Ser Arg Lys Ser Lys Gln His Gln Ser Ser Val Arg Asn Val Ser Thr
                805                 810                 815

tgt gag gtg acc tct tca cag gct atc agc tcc cct cag cga tcc aag      2496
Cys Glu Val Thr Ser Ser Gln Ala Ile Ser Ser Pro Gln Arg Ser Lys
            820                 825                 830

cgt gtc aag gag aac act ccc cca cgg tgc gcc atg gta cac agc agc      2544
Arg Val Lys Glu Asn Thr Pro Pro Arg Cys Ala Met Val His Ser Ser
        835                 840                 845

cca gct tgc agc acc tca gtc acc tgt ggg tgg ggc gac gtg gcc tcc      2592
Pro Ala Cys Ser Thr Ser Val Thr Cys Gly Trp Gly Asp Val Ala Ser

850                 855                 860

agc acc acc cgg gag cga cag cgg cag acg att gtc atc ccc gac acc      2640
Ser Thr Thr Arg Glu Arg Gln Arg Gln Thr Ile Val Ile Pro Asp Thr
865                 870                 875                 880

ccg agc ccc aca gtc agt gtc atc acc atc agc agt gac acc gat gaa      2688
Pro Ser Pro Thr Val Ser Val Ile Thr Ile Ser Ser Asp Thr Asp Glu
                885                 890                 895

gaa gag gag cag aag cac gcc ccc acc agc aca gtc tcc aag caa aga      2736
Glu Glu Glu Gln Lys His Ala Pro Thr Ser Thr Val Ser Lys Gln Arg
            900                 905                 910

aaa aat gtc atc agc tgt gtc acc gtc cac gac tct ccc tac tct gac      2784
Lys Asn Val Ile Ser Cys Val Thr Val His Asp Ser Pro Tyr Ser Asp
        915                 920                 925

tcc tcc agc aac acc agc ccc tac tcg gtg cag cag cgc aca ggg cac      2832
Ser Ser Ser Asn Thr Ser Pro Tyr Ser Val Gln Gln Arg Thr Gly His
    930                 935                 940

aac ggc acc aac acc ttg gac acc aag ggg ggc cta gag aat cac tgc      2880
Asn Gly Thr Asn Thr Leu Asp Thr Lys Gly Gly Leu Glu Asn His Cys
945                 950                 955                 960

act ggc aac ccc cgc acc atc atc gta ccc cca ctg aag acc cag gcc      2928
Thr Gly Asn Pro Arg Thr Ile Ile Val Pro Pro Leu Lys Thr Gln Ala
                965                 970                 975

agt gaa gtg ttg gta gaa tgt gac agc cta ggg cca gcg atc agt gcc      2976
Ser Glu Val Leu Val Glu Cys Asp Ser Leu Gly Pro Ala Ile Ser Ala
            980                 985                 990

agt cac cat tcg tcc tcc ttc aag  tcc aag tcc tcc agc  acc gtg acc      3024
Ser His His Ser Ser Ser Phe Lys  Ser Lys Ser Ser Ser  Thr Val Thr
        995                 1000                1005

tcc acc  agt gga cac tct tca  ggg agc tct tca gga  gcc atc gcc      3069
Ser Thr  Ser Gly His Ser Ser  Gly Ser Ser Ser Gly  Ala Ile Ala
    1010                 1015                 1020

tac cgt  cag cag cgg cca ggc  ccc cac ttc cag cag  cag cag ccc      3114
Tyr Arg  Gln Gln Arg Pro Gly  Pro His Phe Gln Gln  Gln Gln Pro
    1025                 1030                 1035

ctc aat  ctc agc cag gcc cag  cag cac atg gct gcg  gac cgc acc      3159
Leu Asn  Leu Ser Gln Ala Gln  Gln His Met Ala Ala  Asp Arg Thr
    1040                 1045                 1050

ggg agt  cac cgt cga cag cag  gcc tac atc act ccg  acc atg gcg      3204
Gly Ser  His Arg Arg Gln Gln  Ala Tyr Ile Thr Pro  Thr Met Ala
    1055                 1060                 1065

caa gct  ccg tac acc ttc ccg  cac aac agc ccc agc  cat ggc act      3249
Gln Ala  Pro Tyr Thr Phe Pro  His Asn Ser Pro Ser  His Gly Thr
    1070                 1075                 1080

gtt cac  ccc cac ctg gct gcg  gct gcc cac ctc ccc  acc cag cct      3294
Val His  Pro His Leu Ala Ala  Ala Ala His Leu Pro  Thr Gln Pro
    1085                 1090                 1095

cac ctc tac acc tat aca gcg ccc aca gcc cta ggc tcc acc ggc 3339
His Leu Tyr Thr Tyr Thr Ala Pro Thr Ala Leu Gly Ser Thr Gly
    1100                1105                1110

act gta gcc cac ctg gtg gca tcc caa ggc tca gca cgc cac acc 3384
Thr Val Ala His Leu Val Ala Ser Gln Gly Ser Ala Arg His Thr
    1115                1120                1125

gtg cag cac gct gcc tat ccg gcc agc atc gtc cac cag gtc cct 3429
Val Gln His Ala Ala Tyr Pro Ala Ser Ile Val His Gln Val Pro
    1130                1135                1140

gtg agc atg ggg ccc cgg gtc ctg ccc tcg ccc acc atc cac ccc 3474
Val Ser Met Gly Pro Arg Val Leu Pro Ser Pro Thr Ile His Pro
    1145                1150                1155

agt cag tat cca gcc cag ttt gcc cac cag acc tat atc agc gcc 3519
Ser Gln Tyr Pro Ala Gln Phe Ala His Gln Thr Tyr Ile Ser Ala
    1160                1165                1170

tcg cca gcc tcc acc gtc tac act gga tac cca ctg agt cct gcc 3564
Ser Pro Ala Ser Thr Val Tyr Thr Gly Tyr Pro Leu Ser Pro Ala
    1175                1180                1185

aag gtc aac caa tac cct tac ata taa 3591
Lys Val Asn Gln Tyr Pro Tyr Ile
    1190                1195

<210> 8

<211> 1196

<212> PRT

<213> Mus musculus

<400> 8

Met Ala Pro Val Tyr Glu Gly Met Ala Ser His Val Gln Val Phe Ser
1               5                   10                  15

Pro His Thr Leu Gln Ser Ser Ala Phe Cys Ser Val Lys Lys Leu Lys
            20                  25                  30

Val Glu Pro Ser Ser Asn Trp Asp Met Thr Gly Tyr Gly Ser His Ser
        35                  40                  45

Lys Val Tyr Ser Gln Ser Lys Asn Ile Pro Pro Ser Gln Pro Ala Ser
    50                  55                  60

Thr Thr Val Ser Thr Ser Leu Pro Ile Pro Asn Pro Ser Leu Pro Tyr
65                  70                  75                  80

Glu Gln Thr Ile Ile Phe Pro Gly Ser Thr Gly His Ile Val Val Thr
                85                  90                  95

Ser Ala Ser Ser Thr Ser Val Thr Gly Gln Val Leu Gly Gly Pro His
            100                 105                 110

Asn Leu Met Arg Arg Ser Thr Val Ser Leu Leu Asp Thr Tyr Gln Lys
        115                 120                 125

Cys Gly Leu Lys Arg Lys Ser Glu Glu Ile Glu Asn Thr Ser Ser Val
    130                 135                 140

Gln Ile Ile Glu Glu His Pro Pro Met Ile Gln Asn Asn Ala Ser Gly
145                 150                 155                 160

Ala Thr Val Ala Thr Ala Thr Thr Ser Thr Ala Thr Ser Lys Asn Ser
                165                 170                 175

Gly Ser Asn Ser Glu Gly Asp Tyr Gln Leu Val Gln His Glu Val Leu
            180                 185                 190

Cys Ser Met Thr Asn Thr Tyr Glu Val Leu Glu Phe Leu Gly Arg Gly
        195                 200                 205

Thr Phe Gly Gln Val Val Lys Cys Trp Lys Arg Gly Thr Asn Glu Ile
    210                 215                 220

Val Ala Ile Lys Ile Leu Lys Asn His Pro Ser Tyr Ala Arg Gln Gly
225                 230                 235                 240

Gln Ile Glu Val Ser Ile Leu Ala Arg Leu Ser Thr Glu Ser Ala Asp
                245                 250                 255

Asp Tyr Asn Phe Val Arg Ala Tyr Glu Cys Phe Gln His Lys Asn His
            260                 265                 270

Thr Cys Leu Val Phe Glu Met Leu Glu Gln Asn Leu Tyr Asp Phe Leu
        275                 280                 285

Lys Gln Asn Lys Phe Ser Pro Leu Pro Leu Lys Tyr Ile Arg Pro Val
    290                 295                 300

Leu Gln Gln Val Ala Thr Ala Leu Met Lys Leu Lys Ser Leu Gly Leu
305                 310                 315                 320

Ile His Ala Asp Leu Lys Pro Glu Asn Ile Met Leu Val Asp Pro Ser
                325                 330                 335

Arg Gln Pro Tyr Arg Val Lys Val Ile Asp Phe Gly Ser Ala Ser His
            340                 345                 350

Val Ser Lys Ala Val Cys Ser Thr Tyr Leu Gln Ser Arg Tyr Tyr Arg
        355                 360                 365

Ala Pro Glu Ile Ile Leu Gly Leu Pro Phe Cys Glu Ala Ile Asp Met
    370                 375                 380

Trp Ser Leu Gly Cys Val Ile Ala Glu Leu Phe Leu Gly Trp Pro Leu
385                 390                 395                 400

Tyr Pro Gly Ala Ser Glu Tyr Asp Gln Ile Arg Tyr Ile Ser Gln Thr
                405                 410                 415

Gln Gly Leu Pro Ala Glu Tyr Leu Leu Ser Ala Gly Thr Lys Thr Thr
            420                 425                 430

Arg Phe Phe Asn Arg Asp Thr Asp Ser Pro Tyr Pro Leu Trp Arg Leu
        435                 440                 445

Lys Thr Pro Asp Asp His Glu Ala Glu Thr Gly Ile Lys Ser Lys Glu
    450                 455                 460

Ala Arg Lys Tyr Ile Phe Asn Cys Leu Asp Asp Met Ala Gln Val Asn
465                 470                 475                 480

Met Thr Thr Asp Leu Glu Gly Ser Asp Met Leu Val Glu Lys Ala Asp
                485                 490                 495

Arg Arg Glu Phe Ile Asp Leu Leu Lys Lys Met Leu Thr Ile Asp Ala
            500                 505                 510

Asp Lys Arg Val Thr Pro Ile Glu Thr Leu Asn His Pro Phe Val Thr
        515                 520                 525

Met Thr His Leu Leu Asp Phe Pro His Ser Ala His Val Lys Ser Cys
    530                 535                 540

Phe Gln Asn Met Glu Ile Cys Lys Arg Arg Val Asn Met Tyr Asp Thr
545                 550                 555                 560

Val Asn Gln Ser Lys Thr Pro Phe Ile Thr His Val Ala Pro Ser Thr

                565                 570                 575

Ser Thr Asn Leu Thr Met Thr Phe Asn Asn Gln Leu Thr Thr Val His
            580                 585                 590

Asn Gln Ala Pro Thr Thr Ser Ser Ala Thr Leu Ser Leu Ala Asn Pro
        595                 600                 605

Glu Val Ser Ile Leu Asn Tyr Gln Ser Ala Leu Tyr Gln Pro Ser Ala
    610                 615                 620

Ala Ser Met Ala Ala Val Ala Pro Arg Ser Met Pro Leu Gln Thr Gly
625                 630                 635                 640

Thr Ala Gln Ile Cys Ala Arg Pro Asp Pro Phe Gln Gln Ala Leu Ile
                645                 650                 655

Val Cys Pro Pro Gly Phe Gln Gly Leu Gln Ala Ser Pro Ser Lys His
            660                 665                 670

Ala Gly Tyr Ser Val Arg Met Glu Asn Ala Val Pro Ile Val Thr Gln
        675                 680                 685

Ala Pro Gly Ala Gln Pro Leu Gln Ile Gln Pro Gly Leu Leu Ala Gln
    690                 695                 700

Gln Ala Trp Pro Gly Gly Ala Gln Gln Ile Leu Leu Pro Pro Ala Trp
705                 710                 715                 720

Gln Gln Leu Thr Gly Val Ala Thr His Thr Ser Val Gln His Ala Ala
                725                 730                 735

Val Ile Pro Glu Thr Met Ala Gly Thr Gln Gln Leu Ala Asp Trp Arg
            740                 745                 750

Asn Thr His Ala His Gly Ser His Tyr Asn Pro Ile Met Gln Gln Pro
        755                 760                 765

Ala Leu Leu Thr Gly His Val Thr Leu Pro Ala Ala Gln Pro Leu Asn
    770                 775                 780

Val Gly Val Ala His Val Met Arg Gln Gln Pro Thr Ser Thr Thr Ser
785                 790                 795                 800

Ser Arg Lys Ser Lys Gln His Gln Ser Ser Val Arg Asn Val Ser Thr
                805                 810                 815

Cys Glu Val Thr Ser Ser Gln Ala Ile Ser Ser Pro Gln Arg Ser Lys
            820                 825                 830

Arg Val Lys Glu Asn Thr Pro Pro Arg Cys Ala Met Val His Ser Ser
        835                 840                 845

Pro Ala Cys Ser Thr Ser Val Thr Cys Gly Trp Gly Asp Val Ala Ser
    850                 855                 860

Ser Thr Thr Arg Glu Arg Gln Arg Gln Thr Ile Val Ile Pro Asp Thr
865                 870                 875                 880

Pro Ser Pro Thr Val Ser Val Ile Thr Ile Ser Ser Asp Thr Asp Glu
                885                 890                 895

Glu Glu Glu Gln Lys His Ala Pro Thr Ser Thr Val Ser Lys Gln Arg
            900                 905                 910

Lys Asn Val Ile Ser Cys Val Thr Val His Asp Ser Pro Tyr Ser Asp
        915                 920                 925

Ser Ser Ser Asn Thr Ser Pro Tyr Ser Val Gln Gln Arg Thr Gly His
    930                 935                 940

Asn Gly Thr Asn Thr Leu Asp Thr Lys Gly Gly Leu Glu Asn His Cys
945                 950                 955                 960

Thr Gly Asn Pro Arg Thr Ile Ile Val Pro Pro Leu Lys Thr Gln Ala
                965                 970                 975

Ser Glu Val Leu Val Glu Cys Asp Ser Leu Gly Pro Ala Ile Ser Ala
            980                 985                 990

Ser His His Ser Ser Ser Phe Lys  Ser Lys Ser Ser Ser  Thr Val Thr
        995                 1000                 1005

Ser Thr  Ser Gly His Ser Ser  Gly Ser Ser Ser Gly  Ala Ile Ala
    1010                 1015                 1020

Tyr Arg  Gln Gln Arg Pro Gly  Pro His Phe Gln Gln  Gln Gln Pro
    1025                 1030                 1035

Leu Asn  Leu Ser Gln Ala Gln  Gln His Met Ala Ala  Asp Arg Thr
    1040                 1045                 1050

Gly Ser His Arg Arg Gln Gln Ala Tyr Ile Thr Pro Thr Met Ala
    1055                1060                1065

Gln Ala Pro Tyr Thr Phe Pro His Asn Ser Pro Ser His Gly Thr
    1070                1075                1080

Val His Pro His Leu Ala Ala Ala Ala His Leu Pro Thr Gln Pro
    1085                1090                1095

His Leu Tyr Thr Tyr Thr Ala Pro Thr Ala Leu Gly Ser Thr Gly
    1100                1105                1110

Thr Val Ala His Leu Val Ala Ser Gln Gly Ser Ala Arg His Thr
    1115                1120                1125

Val Gln His Ala Ala Tyr Pro Ala Ser Ile Val His Gln Val Pro
    1130                1135                1140

Val Ser Met Gly Pro Arg Val Leu Pro Ser Pro Thr Ile His Pro
    1145                1150                1155

Ser Gln Tyr Pro Ala Gln Phe Ala His Gln Thr Tyr Ile Ser Ala
    1160                1165                1170

Ser Pro Ala Ser Thr Val Tyr Thr Gly Tyr Pro Leu Ser Pro Ala
    1175                1180                1185

Lys Val Asn Gln Tyr Pro Tyr Ile
    1190                1195

<210>  9

<211>  486

<212>  DNA

<213>  human herpesvirus 1

<220>

<221>  CDS

<222>  (1)..(486)

<223>

<400> 9
cta tac aga ccc gcg agc cgt acg tgg ttc gcg ggg ggt gcg tgg ggt 48
Leu Tyr Arg Pro Ala Ser Arg Thr Trp Phe Ala Gly Gly Ala Trp Gly
1               5                   10                  15

ccg ggg ctc ccg ggg aga ccg ggg ctc ccg ggg aga ccg ggg ctc cct 96
Pro Gly Leu Pro Gly Arg Pro Gly Leu Pro Gly Arg Pro Gly Leu Pro
            20                  25                  30

ggg aga ccg ggg ttg tcg tgg atc cct ggg gtc acg cgg tac cct ggg 144
Gly Arg Pro Gly Leu Ser Trp Ile Pro Gly Val Thr Arg Tyr Pro Gly
        35                  40                  45

gtc tct ggg agc tcg cgg tac tct ggg ttc cct agg ttc tcg ggg tgg 192
Val Ser Gly Ser Ser Arg Tyr Ser Gly Phe Pro Arg Phe Ser Gly Trp
    50                  55                  60

tcg cgg aac ccg ggg ctc ccg ggg aac acg cgg tgt cct ggg gat tgt 240
Ser Arg Asn Pro Gly Leu Pro Gly Asn Thr Arg Cys Pro Gly Asp Cys
65                  70                  75                  80

tgg cgg tcg gac ggc ttc aga tgg ctt cga gat cgt agt gtc cgc acc 288
Trp Arg Ser Asp Gly Phe Arg Trp Leu Arg Asp Arg Ser Val Arg Thr
                85                  90                  95

gac tcg tag tag acc cga atc tcc aca ttg ccc cgc cgc ttg atc att 336
Asp Ser Thr Arg Ile Ser Thr Leu Pro Arg Arg Leu Ile Ile
            100                 105                 110

atc acc ccg ttg cgg ggg tcc gga gat cat gcg cgg gtg tcc tcg agg 384
Ile Thr Pro Leu Arg Gly Ser Gly Asp His Ala Arg Val Ser Ser Arg
                115                 120                 125

tgc gtg aac acc tct ggg gtg cat gcc ggc gga cgg cac gcc ttt taa 432
Cys Val Asn Thr Ser Gly Val His Ala Gly Gly Arg His Ala Phe
            130                 135                 140

gta aac atc tgg gtc gcc cgg ccc aac tgg ggc cgg ggg ttg ggt ctg 480
Val Asn Ile Trp Val Ala Arg Pro Asn Trp Gly Arg Gly Leu Gly Leu
            145                 150                 155

gct cat 486
Ala His

<210> 10

<211> 98

<212> PRT

<213> human herpesvirus 1

<400> 10

Leu Tyr Arg Pro Ala Ser Arg Thr Trp Phe Ala Gly Gly Ala Trp Gly
1               5                   10                  15

Pro Gly Leu Pro Gly Arg Pro Gly Leu Pro Gly Arg Pro Gly Leu Pro
            20                  25                  30

Gly Arg Pro Gly Leu Ser Trp Ile Pro Gly Val Thr Arg Tyr Pro Gly
        35                  40                  45

Val Ser Gly Ser Ser Arg Tyr Ser Gly Phe Pro Arg Phe Ser Gly Trp
    50                  55                  60

Ser Arg Asn Pro Gly Leu Pro Gly Asn Thr Arg Cys Pro Gly Asp Cys
65                  70                  75                  80

Trp Arg Ser Asp Gly Phe Arg Trp Leu Arg Asp Arg Ser Val Arg Thr
                85                  90                  95

Asp Ser

<210>  11

<211>  43

<212>  PRT

<213>  human herpesvirus 1

<400>  11

Thr Arg Ile Ser Thr Leu Pro Arg Arg Leu Ile Ile Ile Thr Pro Leu
1               5                   10                  15

Arg Gly Ser Gly Asp His Ala Arg Val Ser Ser Arg Cys Val Asn Thr
            20                  25                  30

Ser Gly Val His Ala Gly Gly Arg His Ala Phe
        35                  40

<210>  12

<211>  18

<212>  PRT

<213>  human herpesvirus 1

<400>  12

Val Asn Ile Trp Val Ala Arg Pro Asn Trp Gly Arg Gly Leu Gly Leu
1               5                   10                  15

Ala His

<210>  13

<211>  456

<212>  DNA

<213>  human herpesvirus 2

<220>

<221>  CDS

<222>  (1)..(456)

<223>

<400>  13
cta ggc aag ccc gcg ggt tgc gcg ggg ttc ccg tgg ggg tct agg ctc      48
Leu Gly Lys Pro Ala Gly Cys Ala Gly Phe Pro Trp Gly Ser Arg Leu
1               5                   10                  15

cct ggg cac ccg tgg ggg tcg tgg ggg tcg cgg gtc cct ggg tat gcg      96
Pro Gly His Pro Trp Gly Ser Trp Gly Ser Arg Val Pro Gly Tyr Ala
            20                  25                  30

cgg gac cct ggg ttc tct ggg aga tcg tgg aac tcg cgg ttc cct ggg      144
Arg Asp Pro Gly Phe Ser Gly Arg Ser Trp Asn Ser Arg Phe Pro Gly
        35                  40                  45

ctc tcg ggg aac ccg ggg ctc cct ggg gac acg tgg tgc cct ggg aat      192
Leu Ser Gly Asn Pro Gly Leu Pro Gly Asp Thr Trp Cys Pro Gly Asn
    50                  55                  60

tct tga tgg tcg gac ggc ttc aga tgg ctt cgg gat cga gag ggc cgc      240
Ser     Trp Ser Asp Gly Phe Arg Trp Leu Arg Asp Arg Glu Gly Arg
65                      70                  75

aca gac tcg tag tag acc cga atc tcc acg ttt ccc cgc cgc cgg atc      288
Thr Asp Ser         Thr Arg Ile Ser Thr Phe Pro Arg Arg Arg Ile
80                          85                  90

atg gtc gcc gcc ccg gtg cgg ggg ccc gtc ggt cgg aag cga gtg ccc      336
Met Val Ala Ala Pro Val Arg Gly Pro Val Gly Arg Lys Arg Val Pro
    95                  100                 105

ttc aag cgt gtc cgc tcc tct ggg ctg cat gcc gtc gga tgg ggt gcc      384
Phe Lys Arg Val Arg Ser Ser Gly Leu His Ala Val Gly Trp Gly Ala
110                 115                 120                 125

ttt taa gga aag gtc tcg gct gcc cgc ccc aac cgg ggt ttg ggg gtg          432
Phe     Gly Lys Val Ser Ala Ala Arg Pro Asn Arg Gly Leu Gly Val
                    130                 135                 140

ggc cgg gga aac ccc gga tgc cat                                          456
Gly Arg Gly Asn Pro Gly Cys His
                145

<210>  14

<211>  65

<212>  PRT

<213>  human herpesvirus 2

<400>  14

Leu Gly Lys Pro Ala Gly Cys Ala Gly Phe Pro Trp Gly Ser Arg Leu
1               5                   10                  15

Pro Gly His Pro Trp Gly Ser Trp Gly Ser Arg Val Pro Gly Tyr Ala
            20                  25                  30

Arg Asp Pro Gly Phe Ser Gly Arg Ser Trp Asn Ser Arg Phe Pro Gly
        35                  40                  45

Leu Ser Gly Asn Pro Gly Leu Pro Gly Asp Thr Trp Cys Pro Gly Asn
    50                  55                  60

Ser
65

<210>  15

<211>  17

<212>  PRT

<213>  human herpesvirus 2

<400>  15

Trp Ser Asp Gly Phe Arg Trp Leu Arg Asp Arg Glu Gly Arg Thr Asp
1               5                   10                  15

Ser

<210> 16

<211> 44

<212> PRT

<213> human herpesvirus 2

<400> 16

Thr Arg Ile Ser Thr Phe Pro Arg Arg Arg Ile Met Val Ala Ala Pro
1               5                   10                  15

Val Arg Gly Pro Val Gly Arg Lys Arg Val Pro Phe Lys Arg Val Arg
            20                  25                  30

Ser Ser Gly Leu His Ala Val Gly Trp Gly Ala Phe
        35                  40

<210> 17

<211> 22

<212> PRT

<213> human herpesvirus 2

<400> 17

Gly Lys Val Ser Ala Ala Arg Pro Asn Arg Gly Leu Gly Val Gly Arg
1               5                   10                  15

Gly Asn Pro Gly Cys His
            20

<210> 18

<211> 18

<212> DNA

<213> Artificial : primer

<400> 18
tacgaaggta tggcctca                                                    18

<210> 19

<211> 22

<212>  DNA

<213>  Artificial : primer

<400>  19
attggtagtt tagtatgcag ac                                              22

<210>  20

<211>  20

<212>  DNA

<213>  Artificial : primer

<400>  20
aaagaagatg ctgaccatcg                                                 20

<210>  21

<211>  29

<212>  DNA

<213>  Artificial : primer

<400>  21
aaacatatgg gcctcctcca gtgtttata                                       29

<210>  22

<211>  19

<212>  DNA

<213>  Artificial : primer

<400>  22
gagtttgagc agatgttta                                                  19

<210>  23

<211>  18

<212>  DNA

<213>  Artificial : primer

<400>  23
ggaagatctg tgaattcg                                                    18

<210>  24

<211>  17

<212>  DNA

<213>  Artificial : primer

<400>  24
atgcggcggc gttattc                                                     17

<210>  25

<211>  16

<212>  DNA

<213>  Artificial : primer

<400>  25
gcgcgggcgg tgtgta                                                      16

<210>  26

<211>  20

<212>  DNA

<213>  Artificial : primer

<400>  26
gtcaccatga cacacctgct                                                  20

<210>  27

<211>  20

<212>  DNA

<213>  Artificial : primer

<400>  27
agggggacac acgatgagag                                                  20

## Description

Homeodomain-interacting protein kinases (HIPKs) possess a kinase domain that display strong sequence similarity with those of other protein kinases belonging to the family of dual specificity Yak1 related kinases (DYRK). One main feature of DYRKs is their dual ability to autophosphorylate on tyrosine residues and to phosphorylate their substrates on serine and threonine residues (Matsuo et al., 2001; Hofmann et al., 2000). However, although HIPKs are able to autophosphorylate and to phosphorylate their substrates on serine and threonine residues, their phosphorylation on tyrosine residues might be mediated by so far uncharacterized kinases (Hofmann et al., 2000 ; Pierantoni et al., 2001). As such, HIPKs constitute a subfamily of DYRKs composed of at least five members: HIPK1, HIPK2, HIPK3, STANK and PKM (Kim et al., 1998 ; Wang et al., 2000). At present, HIPK2 is one of the best characterized member of the HIPK family.

In addition to its kinase domain, HIPK2 contains a domain required for the interaction with several NK homeoproteins, a domain rich in proline, glutamic acid, serine and threonine (PEST) and a domain rich in tyrosine and histidine. The function of these two latter domains is not yet determined although it has been shown that PEST domains may be involved in the stability control of proteins (Rogers et al., 1986). HIPK2 is localized mainly in nuclei and concentrates within a variable numbers of small nuclear domains. The targeting of HIPK2 into these domains is conditioned by its Ubc9-mediated post-translational modification with the ubiquitin-like protein SUMO-1 (Kim et al., 1999). Under certain physiological conditions, HIPK2 is recruited to nuclear domains 10 (ND10) also called PML bodies. This recruitment requires the interaction of HIPK2 with the PML-3 protein (Hofmann et al., 2002).

HIPK2 interacts with several NK homeodomain transcription factors that have important functions during embryonic development and organogenesis. In particular, HIPK2 is a component of a transcriptional corepressor complex containing NK-3, Groucho and a histone deacetylase complex (Kim et al., 1998 ; Choi et al., 1999). HIPK2 regulates the formation and the activity of this complex. It has also been suggested that HIPK2 intervene in the transduction of extracellular signals from the cell surface to the nucleus such as those induced by the tumor necrosis factor (Li et al., 2000 ; Wang et al., 2001a ; Morgan et al., 2002) or those mediated by CD43 and STAT3 (Matsuo et al., 2001 ; Wang et al. 2000). More recently, it has been demonstrated that HIPK2 is a novel activator of the p53 tumor suppressor protein (Hofmann et al., 2002 ; Wang et al., 2001b ; D'Orazi et al., 2002). HIPK2 interacts also with p73 a member of the p53 family and regulates progression through the cell cycle (Pierantoni et al., 2001 ; Kim et al., 2002).

The genome of HSV-1 contains at least 79 genes among 42 of which are dispensable for production of infectious viral particles in cell cultures. Because these dispensable genes should be nevertheless expected to play very important roles in virus-host interactions *in vivo,* many studies have been performed these last decades to elucidate their functions.

US11 was initially described as one of these so-called non-essential genes (Longnecker et al., 1986 ; Brown et al., 1987). US11 protein is among the most abundant viral proteins present in cells late in infection and is packaged in the tegument of the native virions to be delivered into cells after infection (Bhat et al., 1983 ; Gressner et al., 1974 ; Roller et al., 1996). Soon after infection, US11 protein is found in the cytoplasm, either as heterogeneous oligomers or associated with ribosomes or both (Puvion-Dutilleul, 1987 ; MacLean et al., 1987 ; Diaz et al., 1993). Later during infection, US11 protein accumulates into nucleoli and is also found into RNP fibrills as well as in clusters of interchromatin granules. Retention of US11 within nucleoli and nuclear export are mediated by a unique motif embedded into the C-terminal part of the protein (Catez et al., 2002). In addition, this part of the protein contains a motif that confers to US11 an intercellular trafficking activity (Koshizuka et al., 2001).

Since the first identification of US11 as a DNA-binding protein, strong evidences have been brought showing that this protein possess also RNA-binding activities which appear essential for several of its biological functions, like control of mRNA transport and translation (Diaz et al., 1996 ; Duc et al., 2000), and regulation of PKR activity (Poppers et al., 2000 ; Cassady et al., 1998). Moreover, even though US11 protein is dispensable for viral growth in cell cultures, it plays an important role for the replication of HSV-1 in the adrenal gland, an organ important for viral penetration in the spinal cord and brain (Nishiyama et al., 1993) and in cells submitted to thermal stress (Brown et al., 1987). US11 has also been implicated in the recovery of protein synthesis and survival in heat shock treated cells (Diaz-Latoud et al., 1997).

The inventors have designed a yeast two-hybrid screen of a mouse library using US11 as a bait in order to identify cellular factors susceptible to interact with this protein. This allowed them to isolate a cDNA whose sequence encompassed the PEST domain of the murine HIPK2 protein. Co-immunoprecipitation of US11 with transiently expressed Flag-tagged HIPK2/PEST provided further evidence for their tight association in mammalian cells. Moreover, the observation that some modified forms of US11 containing either a single or a very few amino acids substitutions interacted slightly or do not interacted at all attest for the specificity of this interaction.

Thereby, the inventors demonstrated that US11 interacts with HIPK2 and that this interaction occurs through the PEST domain of HIPK2.

Interestingly, the HIPK2 intracellular distribution was shown to be strongly modified after co-expression of US11. More specifically, US11 completely abolished the dot like pattern of HIPK2 nuclear distribution and seemed to induce an accumulation of this protein within the nucleoplasm. More importantly, colony formation assays demonstrated that the HIPK2/p53 induction of apoptosis was inhibited by US11 indicating that this protein could antagonize HIPK2-induced apoptosis of HSV-1 or HSV-2

The inventors thus identified US11 as a new mediator of the HSV-1 and HSV-2 dependent inhibitions of apoptosis.

### Definitions

A "polynucleotide" or "nucleotide sequence" is a serie of nucleotides (or nucleoside plus phosphate group) in a nucleic acid, such as DNA and RNA, and means any chain of two or more nucleotides. A nucleotide sequence typically carries genetic information, including the information used by cellular machinery to make proteins and enzymes. These terms include double or single stranded genomic and cDNA, RNA, any synthetic and genetically manipulated polynucleotide, and both sense and anti-sense polynucleotide (although only sense strands are being represented herein). This includes single- and double-stranded molecules, *i.e.,* DNA-DNA, DNA-RNA and RNA-RNA hybrids, as well as "protein nucleic acids" (PNA) formed by conjugating bases to an amino acid backbone. This also includes nucleic acids containing modified bases, for example thio-uracil, thio-guanine and fluoro-uracil.

The nucleic acids herein may be flanked by natural regulatory (expression control) sequences, or may be associated with heterologous sequences, including promoters, internal ribosome entry sites (IRES) and other ribosome binding site sequences, enhancers, response elements, suppressors, signal sequences, polyadenylation sequences, introns, 5'- and 3'- non- coding regions, and the like. The nucleic acids may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphoramidate linkage. Furthermore, the polynucleotides herein may also be modified with a label capable of providing a detectable signal, either directly or indirectly. Exemplary labels include radioisotopes, fluorescent molecules, biotin, and the like.

A *"promoter"* or *"promoter sequence"* is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease SI), as well as protein binding domains (consensus sequences) responsible for the binding of RNA polymerase. The promoter may be operatively associated with other expression control sequences, including enhancer and repressor sequences.

Promoters which may be used to control gene expression include, but are not limited to, cytomegalovirus (CMV) promoter (V.S. Patents No. 5,385,839 and No. 5,168,062), the SV40 early promoter region (Benoist and Chambon, 1981), the promoter contained in the 3' long terminal repeat of Rous sarcoma virus (Yamamoto, et al., 1980), the herpes thymidine kinase promoter (Wagner et al., 1981), the regulatory sequences of the metallothionein gene (Brinster et al., 1982) ; prokaryotic expression vectors such as the beta-lactamase promoter (Villa-Komaroff et al., 1978), or the tac promoter (DeBoer et al., 1983); see also "Useful proteins from recombinant bacteria" in Scientific American 1980, 242:74-94; promoter elements from yeast or other fungi such as the Gal 4 promoter, the ADC (alcohol dehydrogenase) promoter, PGK (phosphoglycerol kinase) promoter, alkaline phosphatase promoter; and transcriptional control regions that exhibit hematopoietic tissue specificity, in particular: beta-globin gene control region which is active in myeloid cells (Mogram et al., 1985 ; Kollias et al., 1986), hematopoietic stem cell differentiation factor promoters, erythropoietin receptor promoter (Maouche et al., 1991), etc.

A *"coding sequence"* or a sequence *"encoding"* an expression product, such as a RNA, polypeptide, protein, or enzyme, is a nucleotide sequence that, when expressed, results in the production of that RNA, polypeptide, protein, or enzyme, *i.e.,* the nucleotide sequence encodes an amino acid sequence for that polypeptide, protein or enzyme. A coding sequence for a protein may include a start codon (usually ATG) and a stop codon.

The term *"gene",* also called a *"structural gene*" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription.

A coding sequence is *"under the control of"* or *"operatively associated with"* transcriptional and translational control sequences in a cell when RNA polymerase transcribes the coding sequence into RNA, particularly mRNA, which is then trans-RNA spliced (if it contains introns) and translated into the protein encoded by the coding sequence.

The terms *"vector",* and *"expression vector"* mean the vehicle by which a DNA or RNA sequence (e.g. a foreign gene) can be introduced into a host cell, so as to transform the host and promote expression (e.g. transcription and translation) of the introduced sequence. Vectors include plasmids, phages, viruses, etc.; they are discussed in greater detail below.

Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. A "cassette" refers to a DNA coding sequence or segment of DNA that codes for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a *"DNA construct."* A common type of vector is a *"plasmid",* which generally is a self-contained molecule of double-stranded DNA, usually of bacterial origin, that can readily accept additional (foreign) DNA and which can readily be introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts. Non-limiting examples include pKK plasmids (Clonetech), pUC plasmids, pET plasmids (Novagen, Inc., Madison, WI), pRSET or pREP plasmids (Invitrogen, San Diego, CA), or pMAL plasmids (New England Biolabs, Beverly, MA), and many appropriate host cells, using methods disclosed or cited herein or otherwise known to those skilled in the relevant art. Recombinant cloning vectors will often include one or more replication systems for cloning or expression, one or more markers for selection in the host, e.g. antibiotic resistance, and one or more expression cassettes.

The terms *"express"* and *"expression"* mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, e.g. the resulting protein, may also be said to be *"expressed"* by the cell. An expression product can be characterized as intracellular, extracellular or secreted. The term *"intracellular"* means something that is inside a cell. The term *"extracellular"* means something that is outside a cell. A substance is *"secreted"* by a cell if it appears in significant measure outside the cell, from somewhere on or inside the cell.

The terms *"transfection"* and "transformation" mean the introduction of a foreign nucleic acid into a cell. These terms " mean the introduction of a *"foreign"* (*i.e.* extrinsic or extracellular) gene, DNA or RNA sequence to a host cell, so that the host cell will express the introduced gene or sequence to produce a desired substance, typically a protein or enzyme coded by the introduced gene or sequence. The introduced gene or sequence may also be called a *"cloned"* or *"foreign"* gene or sequence, may include regulatory or control sequences, such as start, stop, promoter, signal, secretion, or other sequences used by a cell's genetic machinery. The gene or sequence may include nonfunctional sequences or sequences with no known function. A host cell that receives and expresses introduced DNA or RNA has been "transfected" or *"transformed"* and is a "transfectant", a *"transformant"* or a "clone." The DNA or RNA introduced to a host cell can come from any source, including cells of the same genus or species as the host cell, or cells of a different genus or species.

The term *"host cell"* means any cell of any organism that is selected, modified, transformed, grown, or used or manipulated in any way, for the production of a substance by the cell, for example the expression by the cell of a gene, a DNA or RNA sequence, a protein or an enzyme. Host cells can further be used for screening or other assays, as described infra.

The term *"expression system"* means a host cell and compatible vector under suitable conditions, e.g. for the expression of a protein coded for by foreign DNA carried by the vector and introduced to the host cell. Common expression systems include *E. coli* host cells and plasmid vectors, insect host cells and Baculovirus vectors, and mammalian host cells and vectors. In a specific embodiment, the protein of interest is expressed in COS-1 or C2C12 cells. Other suitable cells include CHO cells, HeLa cells, 293T (human kidney cells), mouse primary myoblasts, and NIH 3T3 cells.

As used herein, the term *"homologous"* in all its grammatical forms and spelling variations refers to the relationship between proteins that possess a "common evolutionary origin," including proteins from superfamilies (*e.g*., the immunoglobulin superfamily) and homologous proteins from different species (*e.g.*, myosin light chain, etc.) (Reeck et al., 1987). Such proteins (and their encoding genes) have sequence homology, as reflected by their sequence similarity, whether in terms of percent similarity or the presence of specific residues or motifs at conserved positions as may be determined by alignment using, for example, the GCG (Genetics Computer Group, Program Manual for the GCG Package, Version 7, Madison, Wisconsin) pileup program, or any of the programs BLAST, FASTA, etc...

The term *"HIPK2"* denotes the human Homeodomain-interacting protein kinase 2, but also homologous proteins from other mammalian species, such as mice or hamster, and splicing variant thereof. Cloning of human HIPK2 has been reported in Hofmann et al. (2000). mRNA sequences of human HIPK2 and of an alternative splicing variant thereof are deposited in Genbank under accession number NM_022740 (polynucleotide sequence : SEQ ID No:1, amino acid sequence: SEQ ID No:2) and AF207702 (polynucleotide sequence : SEQ ID No:3, amino acid sequence: SEQ ID No:4). Two mouse splicing variants (hereafter called HIPK2a and HIPK2b) have been identified by the inventors of the instant application and the corresponding mRNA and amino acid sequences are deposited in Genbank under accession numbers AF333791 (HIPK2a: polynucleotide sequence SEQ ID No: 5, amino acid sequence: SEQ ID No: 6) and AF333792 (HIPK2b: polynucleotide sequence SEQ ID No: 7, amino acid sequence: SEQ ID No: 8).

As used herein, the term *"PEST"* or *"PEST domain"* is meant for the HIPK2 domain rich in proline, glutamic acid, serine and threonine. For instance, mouse and hamster PEST domains have been defined as consisting of amino acid residues 839 to 934, and 812 to 907, respectively, of HIPK2 protein (Kim et al., 1998, and Kim et al., 2002). Human HIPK2 contains two potential PEST sequences from position 872 to position 901 and position 922 to position 941 (Wang et al. 2001a).

The term *"herpes simplex virus"* (HSV) is intended for viruses from the Alphaherpesvirinae subfamily of herpes viruses that belong to the genus simplexvirus. Examples of HSV include the human herpes simplex virus type 1 (HSV-1) or type 2 (HSV-2).

*"US11"* refers to the Unique Short sequence 11 protein of a herpes simplex virus type 1 and type 2. In the context of the invention, said US11 protein may originate from may be of any specie, genotype and subtype of herpes simplex virus, where appropriate. The term US11 includes in particular US11 from HSV-1 and HSV-2. A HSV-1 coding sequence for US11 is described for instance in the Genbank database under accession number X02138 (polynucleotide sequence: SEQ ID No: 9, amino acid sequence: SEQ ID No: 10). Similarly a HSV-2 coding sequence for US11 is described in the Genbank database under accession number NC_001798 (polynucleotide sequence: SEQ ID No: 11, amino acid sequence: SEQ ID No: 12).

The inventors have shown that US11 and HIPK2 can interact, in particular by way of the PEST domain of HIPK2, to form US11/HIPK2 complexes. The binding of US11 to HIPK2 has been demonstrated by the inventors as implicated in rescuing the cells from the apoptotic process elicited by HIPK2.

Thus, in the context of the present invention, the term *"modulator"* is intended for a molecule that modulates, i.e. antagonizes or promotes, the US11/HIPK2 complex. More specifically, said modulator may act by modulating either the interaction between US11 and HIPK2, or the activity of a US11/HIPK2 complex. Typically, the term modulator encompasses molecules with antagonist and agonist activity.

The term *"antagonist"* is intended for a molecule that may (i) interfere with the formation of, or disrupt, a US11/HIPK2 complex, or (ii) interfere or inhibit the activity of a US11/HIPK2 complex, in particular the apoptosis rescuing activity associated with the US11/HIPK2 complex. In other words, antagonists according to the invention may include molecules capable of preventing or limiting the interaction of US11 with HIPK2, or molecules capable of inducing a dissociation of a US11/HIPK2 complex, partially or totally, where said complex is formed. Examples of antagonists may include for instance an isolated HIK2 PEST domain, or a fragment of HIPK2 comprising the PEST domain, that may act as a competitor with HIPK2 for binding with US11.

By *"agonist",* is meant a molecule that is involved in, or promotes, (i) formation of a US11/HIPK2 complex, or (ii) the activity of a US11/HIPK2 complex.

The term *"candidate molecule"* includes virtually any candidate molecule which may interact with US11 and/or HIPK2 and/or US11/HIPK2 complex. The candidate molecule may be peptide or non-peptide in nature. Any compound isolated from natural sources such as plants, animals or even marine, forest or soil samples, may be assayed, as may any synthetic chemical or recombinant protein.

The term *"herpes simplex virus infection"* denotes the condition of a subject infected with HSV, either in a primary or a latent infectious state, as well as in a reactivation period. Primary infection generally occurs through a break in the mucus membranes of the mouth or throat, via the eye or genitals or directly via minor abrasions in the skin. Initial infection is usually asymptomatic, although there may be minor local vesicular lesions. Local multiplication ensues, followed by viraemia and systemic infection. There then follows life-long latent infection with periodic reactivation. The delicate balance of latency may be upset by various disturbances, physical (injury, U.V, hormones, etc) or psychological (stress, emotional upset). Reactivation of latent virus leads to recurrent disease wherein virus travels back to surface of body and replicates, causing tissue damage. HSV-1 is primarily associated with oral and ocular lesions whereas HSV-2 is primarily associated with genital and anal lesions.

Accordingly, *"treatment of a herpes simplex virus infection"* is meant for the prevention of the onset of a primary infection and/or for the prevention of occurrence of a viral reactivation period in the subject with latent infection, as well as the treatment of the lesions caused by the virus reactivation.

As used herein *"pharmaceutically acceptable carrier"* includes any solvents, dispersion media, coatings, antibacterial and antifungal agents, isotonic and absorption delaying agents and the like that do not produce an adverse, allergic or other untoward reaction when administered to an animal, or a human, as appropriate. The use of such media and agents for pharmaceutical active substances is well known in the art. Except insofar as any conventional media or agent is incompatible with the active ingredient, its use in the therapeutic compositions is contemplated.

According to the invention, the term *"subject"* or *"patient"* is meant for any living specie likely to be infected with a herpes simplex virus, in particular with HSV. Mammals, and in particular human, monkey or bovine are examples of hosts for herpes simplex viruses.

### Protein complexes, compositions and kits

The invention provides an isolated protein complex, further called *"US11*/*HIPK2 complex"* or *"US11*/*HIPK2 protein complex",* comprising at least two interacting proteins, said complex being selected from the group consisting of (a) US11 and HIPK2, (b) US11 and a fragment of HIPK2, (c) a fragment of US11 and HIPK2, and (d) a fragment of US11 and a fragment of HIPK2.

The interacting proteins may be native or recombinant. Recombinant proteins may be produced in eukaryotic or prokaryotic systems according to conventional procedures of molecular biology, microbiology, and recombinant DNA techniques well-known by the one skilled in the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989") ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et al. (eds.), Current Protocols in Molecular Biology, John Wiley & Sons, Inc. (1994).

Advantageously, the interacting proteins can be produced as a fusion protein that may include a peptide linker between the interacting proteins, which may serve to promote the interaction of the proteins.

*A "fragment of HIPK2 protein"* or *"HIPK2 fragment"* is meant for N-terminally and/or C-terminally truncated form of HIPK2 that retains capacity of binding with native US11, or a fragment thereof. Preferably said HIPK2 fragment consists of or comprises the PEST domain.

A *"fragment of US11 protein"* or *"US11 fragment"* is meant for N-terminally and/or C-terminally truncated form of US11 that retains capacity of binding with native HIPK2, or with a fragment thereof such as a fragment containing the HIPK2 PEST domain.

Production of US11 or HIPK2 fragments may be readily achieved by the one skilled in the art. Assaying their capacity to retain binding capacity may be performed by detecting protein interactions with various systems, including the yeast two-hybrid system, affinity chromatography, co-immunoprecipitation, subcellular fractionation isolation of large molecular complexes and proteomics.

According to the above embodiment, the US11 and HIPK2 proteins are provided in the form of a US11/HIPK2 complex. Alternatively, the US11 and HIPK2 proteins may be provided together within a composition, or separately in a kit.

Accordingly the invention further provides a protein composition comprising at least two interacting proteins selected from the group consisting of (a) US11 or a fragment of US11, and (b) HIPK2 or a fragment of HIPK2, optionally in a pharmaceutically acceptable carrier.

The invention also provides a kit comprising (a) US11 or a fragment of US11, and (b) HIPK2 or a fragment of HIPK2.

In the sections below, unless otherwise specified, the term "HIPK2 protein" includes a HIPK2 fragment. Similarly, unless otherwise specified, the term "US11 protein" includes a US11 fragment.

### Vectors and Host cells

The invention also relates to an isolated host cell coexpressing US11 and HIPK2 proteins. In particular a host cell according to the invention comprises, or has been transformed with a US11 expressing vector and optionally a HIPK2 expressing vector.

Since HIPK2 expression may be very low in cells, it may be preferable to overexpress HIPK2, e.g. by transforming the cells with a HIPK2 expressing vector. Otherwise, HIPK2 may be overexpressed and/or activated for instance by exposing a host cell to UVs, or by treating said cell with arsenic (AS₂O₃).

Where appropriate, US11 and HIPK2 polynucleotide sequences may be carried on two separate vectors or on a same vector (for example a vector carrying an International Ribosome Entry Site (IRES) for the expression of the second coding sequence). Another possibility is that both sequences are cloned in frame, under the control of a same promoter so as to ensure coexpression of US11 and HIPK2 proteins. Accordingly a vector comprising a polynucleotide sequence encoding US11 and HIPK2 proteins, in particular in the form of a fusion protein that optionally includes a peptide linker between US11 and HIPK2 proteins, is within the scope of the invention. The choice of a suitable expression system is within the ordinary skills in the art. Preferably, said host cell is a p53 expressing cell such as Hek 293 cells (also named 293T), HCT116, U2OS, and RKO.

### Antibodies against US11/HIPK2 complex

As shown above, US11 interacts with HIPK2 to form a complex. For the purpose of antibody production, a complex of the two proteins may be prepared, *e.g.*, by mixing purified preparations of each of the two proteins. If desired, the protein complex may be stabilized by cross-linking the proteins in the complex, by methods known to those of skill in the art.

Accordingly, the invention proposes an isolated antibodies *"specific"* for the US11/HIPK2 complex, *i.e.* antibodies that are reactive with said complex but that are not reactive with US11 and HIPK2 proteins.

The antibodies of the present invention can be monoclonal or polyclonal antibodies, single chain or double chain, chimeric antibodies, humanized antibodies, or portions of an immunoglobulin molecule, including those portions known in the art as antigen binding fragments Fab, Fab', F(ab')2 and F(v). They can also be immunoconjugated *,e.g.* with a toxine, or labelled antibodies.

Whereas polyclonal antibodies may be used, monoclonal antibodies are preferred for they are more reproducible in the long run.

Procedures for raising *"polyclonal antibodies"* are also well known. Polyclonal antibodies can be obtained from serum of an animal immunized against the US11/HIPK2 complex, which can be produced by genetic engineering for example according to standard methods well-known by one skilled in the art. Typically, such antibodies can be raised by administering the protein complex subcutaneously to New Zealand white rabbits which have first been bled to obtain pre-immune serum. The antigens can be injected at a total volume of 100 µl per site at six different sites. Each injected material will contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed in Harlow et al. (1988) which is hereby incorporated by reference.

A *"monoclonal antibody"* in its various grammatical forms refers to a population of antibody molecules that contain only one specie of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, *e.g.* a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et al., 1988). Monoclonal antibodies (mAbs) may be prepared by immunizing purified US11/HIPK2 protein complex from any of a variety of mammalian species into a mammal, *e.g.* a mouse, rat, human and the like mammal. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody. This standard method of hybridoma culture is described in Kohler and Milstein (1975).

While mAbs can be produced by hybridoma culture, the invention is not to be so limited. Also contemplated is the use of mAbs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No. WO 890099 to Robinson et *al*.; European Patent Publications No. 0239400 to Winter et al. and No. 0125023 to Cabilly et *al.*

Antibody generation techniques not involving immunisation are also contemplated such as for example using phage display technology to examine naive libraries (from non-immunised animals); see Barbas et al. (1992), and Waterhouse et al. (1993).

### Screening methods

The invention relates to a method, in particular *in vitro,* of screening or identifying candidate molecules capable of modulating a US11/HIPK2 complex. Such molecules capable of modulating the US11 and HIPK2 interaction or of modulating the US11/HIPK2 complex activity comprise molecules useful for preventing or treating a herpes simplex virus infection.

More specifically, the method of screening or identifying candidate molecules capable of modulating a US11/HIPK2 complex may comprise the steps consisting of :
a) contacting at least one candidate molecule with a US11/HIPK2 protein complex as described above, and/or with US11 and HIPK2 proteins; and
b) determining the presence or absence of binding of the candidate molecule to said US11/HIPK2 complex, US11 protein or HIPK2 protein;
wherein the binding of the candidate molecule to said US11/HIPK2 complex, US11 protein or HIPK2 protein, is indicative of a modulator of the US11/HIPK2 complex.

According to a first embodiment, said modulator of US11/HIPK2 complex may interfere with or promote the apoptosis rescuing activity associated with the US11/HIPK2 complex, without necessarily disrupting, even partially, said complex. Such a modulator may thus be identified by the above method by specifically detecting binding to the US11/HIPK2 complex. A method of screening or identifying a modulator of US11/HIPK2 complex activity may thus comprise the steps of :
a) contacting at least one candidate molecule with a US11/HIPK2 protein complex as described above; and
b) determining the presence or absence of binding of the candidate molecule to said US11/HIPK2 complex;
wherein the binding of the candidate molecule to said US11/HIPK2 complex is indicative of a modulator of the US11/HIPK2 complex.

Alternatively, the interfering or promoting activity of the modulator of US11/HIPK2 activity may be assayed according to a functional test, such as a cell colony assay. A method of screening or identifying candidate molecules capable of modulating activity of a US11/HIPK2 complex may thus comprise the steps consisting of :
a) contacting a p53 expressing cell containing a HIPK2 expressing vector and a US11 expressing vector with a p53-mediated apoptosis inducer, in the presence or in the absence of a candidate molecule, under conditions sufficient to allow apoptosis to occur where the candidate molecule is absent; and
b) determining the number of cell colonies in the presence and in the absence of the candidate molecule;
wherein an altered number of colonies as estimated in the presence of the candidate molecule, compared with the number of colonies as estimated in the absence of the candidate molecule, is indicative of a molecule capable of modulating activity of a US11/HIPK2 complex.

The principle of this cell colony assay is explained more thoroughly below.

Said modulator may be an antagonist of US11/HIPK2 complex activity. Accordingly, in the above method, a candidate molecule would be identified as an antagonist where a decreased number of colonies is detected, as estimated in the presence of the candidate molecule, compared with the number of colonies as estimated in the absence of the candidate molecule. Antagonists of US11/HIPK2 complex activity should constitute molecules useful for the treatment of a herpes simplex infection.

Alternatively, said modulator may be an agonist of US11/HIPK2 complex activity. In that case, in the above method, a candidate molecule would be identified as an agonist where an increased number of colonies is detected, as estimated in the presence of the candidate molecule, compared with the number of colonies as estimated in the absence of the candidate molecule.

According to a second embodiment, said modulator of US11/HIPK2 complex is a modulator of the interaction of US11 with HIPK2.

The invention more particularly provides a method of screening or identifying candidate molecules wherein a candidate molecule is assayed for its capacity to modulate the US11 and HIPK2 interaction, *i.e,* of preventing or promoting the binding of US11 with HIPK2, or also to dissociate, partly or totally, the so-formed US11/HIPK2 complexes.

According to this embodiment, the invention relates to a method of screening or identifying candidate molecules capable of modulating the interaction of US11 with HIPK2, which method comprises the steps consisting of:
a) contacting at least one candidate molecule with a HIPK2 protein and a US11 protein, under conditions suitable to allow US11 and HIPK2 to interact in the absence of the candidate molecule; and
b) determining the interaction of US11 and HIPK2 in the absence and in the presence of the candidate molecule;
wherein a modulation of the interaction of US11 and HIPK2 is indicative of a molecule capable of modulating the US11 and HIPK2 interaction.

Modulators of US11 and HIPK2 interaction include agonists and antagonists of US11/HIPK2 interaction

Preferably, the invention relates to method of screening molecules capable of modulating the US11 and HIPK2 interaction, and in particular of molecules useful for preventing or treating a herpes simplex virus infection, wherein an antagonist of the US11/HIPK2 interaction is identified. The candidate molecule may be assayed for its capacity to interfere with or disrupt the interaction of US11 with HIPK2. Said method of screening thus comprises the steps consisting of :
a) contacting at least one candidate molecule with a HIPK2 protein and a US11 protein, under conditions suitable to allow HIPK2 and US11 to interact in the absence of the candidate molecule; and
b) determining the interaction of HIPK2 and US11 in the absence and in the presence of the candidate molecule;
wherein a decreased interaction of US11 and HIPK2 as measured in the presence of the candidate molecule, compared with HIPK2 and US11 interaction as measured in the absence of the candidate molecule, is indicative of an antagonist of US11/HIPK2 interaction.

Alternatively, the invention relates to method of identifying an agonist of US11/HIPK2 interaction, said method comprising the steps consisting of :
a) contacting at least one candidate molecule with a HIPK2 protein and a US11 protein, under conditions suitable to allow HIPK2 and US11 to interact in the absence of the candidate molecule; and
b) determining the interaction of HIPK2 and US11 in the absence and in the presence of the candidate molecule;
wherein a increased interaction of US11 and HIPK2 as measured in the presence of the candidate molecule, compared with HIPK2 and US11 interaction as measured in the absence of the candidate molecule, is indicative of an agonist of the US11/HIPK2 interaction.

Advantageously, a US11 protein, a HIPK2 protein and at least one candidate molecule are contacted and incubated under conditions suitable to allow them to interact, and in particular to form or dissociate US11/HIPK2 complexes.

Alternatively, at least one candidate molecule and a US11 protein or a HIPK2 protein are contacted under conditions suitable to allow them to optionally interact. The missing US11 protein or HIPK2 protein is further added and the three components are contacted under conditions suitable to allow them to interact, and optionally to form US11/HIPK2 complexes.

Still alternatively, a US11 protein and a HIPK2 protein are contacted under conditions suitable to formation of US11/HIPK2 complexes. At least one candidate molecule is further added and the three components are contacted under conditions suitable to allow them to interact, and optionally to allow US11/HIPK2 complexes to dissociate.

Determining of the capacity of the candidate molecule to modulate the interaction of US11 with HIPK2, or to bind and modulate a US11/HIPK2 complex, can be achieved by any appropriate mean well-known by the one skilled in the art, such as classical separation and/or detection methods. For instance, US11/HIPK2 complexes may be quantified in the presence of increasing concentrations of candidate molecules. US11/HIPK2 binding can be carried out using classical analysis procedures such as *in vitro* assays of protein binding, *e.g.,* wherein one or both of the US11 or HIPK2 components are attached to a detectable label, and/or are immobilized may be employed. In general, methods classically used for the detection of a positive protein-protein interaction, such as two-hybrid system, affinity chromatography, co-immunoprecipitation, subcellular fractionation and isolation of large molecular complexes, immunoblotting, or immunolabelling, optionally in association with detectable markers such as fluorescent, isotopic or chromogenic labelling, can also be used to identify agents that interfere or disrupt said interaction. As an example, cells transfected with DNAs coding for US11 and HIPK2 can be treated with various candidate molecules, and co-immunoprecipitations can be performed. Alternatively, a derivative of the yeast two-hybrid system, called the reverse yeast two-hybrid system (Leanna and Hannink, 1996), can be used, provided that the two proteins interact in the straight yeast two-hybrid system. Reference can further be made to Current Protocols in Molecular Biology, Edited by F.M. Ausubel et al. (John Wiley and Sons).

Advantageously, the capacity of the candidate molecule to modulate the US11/HIPK2 complex may be achieved according to a functional test such as the colony formation assay described below. Briefly, p53 expressing cells, such as Hek 293 cells, may be transfected with a HIPK2 expressing vector, preferably allowing also the expression of an antibiotic resistance gene (such as Neo^{R}) so as to make it possible to select the transformed cells, together with an expression vector coding for US11. After the beginning of transfection, cells may be selected for resistance to the appropriate antibiotic (such as Geneticin (G418) for Neo^{R}) in the presence or not of a candidate molecule, and the number of colonies estimated for each experimental condition.

Exposure to the antibiotic further forces the cells to express HIPK2 and thus to undergo apoptosis.

Cell apoptosis may be amplified by exposing cells to UVs or contacting cells with IFN-α, so as to enhance HIPK2 expression and thereby induce apoptosis, in particular p53 mediated apoptosis.

In such a test, a decreased number of colonies as estimated in the presence of the candidate molecule, compared with the number of colonies as estimated in the absence of the candidate molecule, which is indicative of a candidate molecule that promotes cell apoptosis and thus of an antagonist of the US11/HIPK2 interaction or of US11/HIPK2 complex.

Conversely, an increased number of colonies as estimated in the presence of the candidate molecule, compared with the number of colonies as estimated in the absence of the candidate molecule, which is indicative of a candidate molecule that rescues cells from apoptosis and thus of an agonist of the US11/HIPK2 interaction or of US11/HIPK2 complex.

Accordingly, the invention further provides an *in vitro* method of screening molecules useful for preventing or treating a human herpes simplex virus infection, wherein a candidate molecule is assayed for its capacity to interfere with US11-mediated inhibition of cellular apoptosis, and in particular with the apoptosis rescuing activity associated with the US11/HIPK2 complex.

More specifically, said method may comprises the steps consisting of :
a) cultivating a cell, in particular a p53 expressing cell containing a HIPK2 expressing vector and a US11 expressing vector, in the presence or in the absence of a candidate molecule, under conditions sufficient to allow HIPK2 expression and cell apoptosis to occur where the candidate molecule is absent; b) determining the number of cell colonies in the presence and in the absence of the candidate molecule;
wherein a decreased number of colonies as estimated in the presence of the candidate molecule, compared with the number of colonies as estimated in the absence of the candidate molecule, is indicative of a molecule useful for preventing or treating a herpes simplex virus infection.

US11 and HIPK2 coding sequences may be carried on two separate vectors or on a same vector (for example a vector carrying an International Ribosome Entry Site (IRES) for the expression of the second coding sequence). Another possibility is that both sequences are cloned in frame, under the control of a same promoter so as to ensure co-expression of US11 and HIPK2 proteins.

The p53 expressing cell may for instance be selected from the group consisting of Hek 293 cells (293T) HCT116, U2OS, and RKO. However, any p53 expressing cell may be suitable to carry out the above method.

According to said method the cultivated cell may be further exposed to UVs and/or contacted with IFN-α so as to enhance HIPK2 expression.

### Therapeutical compositions

The inventors have demonstrated that US11 is a mediator of the HSV-1 dependent inhibition of cellular apoptosis. Without willing to be linked to a particular mechanism of action, US11 could prevent cells infected with HSV-1 or HSV-2 to undergo apoptosis during the reactivation and infection phases of the virus, and in particular following UV exposure. This inhibition of cell apoptosis would involve US11 binding to HIPK2 and thereby prevention of p53 activation by HIPK2. Inhibition of cellular apoptosis by US11 should thus make it possible for reactivated HSV-1 or HSV-2 to replicate in the cells rescued from apoptosis.

As a consequence, an antagonist of the US11/HIPK2 complex, *i.e.* a molecule capable of interfering with the formation and/or functioning of said complex, represents a molecule useful for the treatment of HSV-1 or HSV-2 infection, more specifically for preventing the virus to replicate under conditions of physical or psychological disturbance, such as UV exposure.

The invention thus provides a composition comprising a modulator of US11/HIPK2 complex, in particular an antagonist of US11/HIPK2 complex, in a pharmaceutically acceptable carrier.

According to a first embodiment, said modulator of US11/HIPK2 complex is an antagonist that interferes specifically with the interaction of US11 with HIPK2. As used herein, the term *"specifically"* or *"specific"* is used to denote an antagonist of US11/HIPK2 interaction, or a modulator of US11/HIPK2 complex, that does not interfere with or inhibit US11 and/or HIPK2 activity.

Agents capable of interfering with US11 binding to HIPK2 interaction or of modulating the US11/HIPK2 complex may be identified by a screening method as described above. Such agents include for instance peptides, antibodies, aptamers, or non peptide small molecules.

Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as *E. coli* Thioredoxin A that are selected from combinatorial libraries by two hybrid methods (Colas et al., 1996).

According to a second embodiment, said modulator of US11/HIPK2 complex is an antagonist of US11/HIPK2 complex activity, such as can be identified by a screening method as described above.

### Therapeutical applications

As discussed above, the inventors have demonstrated that US11 is involved in the protection of HSV infected cells from the p53-mediated apoptosis that is induced by HIPK2, for instance upon UV exposure. These results identify the US11/HIPK2 complex, but also the US11 and HIPK2 proteins, as new targets for the treatment of a human herpes simplex virus (HSV) infection.

Actually, any mean promoting the HIPK2 apoptosis-inducing activity, or interfering with the US11 inhibition of apoptosis, should be useful for treatment of a HSV-1 or HSV-2 infection. This includes:
- an antagonist of US11/HIPK2 complex, *i.e.* an antagonist of the US11/HIPK2 interaction and/or complex activity, preferably a molecule interfering with the apoptosis rescuing activity associated with the US11/HIPK2 complex;
- promoting HIPK2-induced apoptosis, for instance by administering a HIPK2 agonist; and
- interfering with US11 activity, for instance by administering a US11 antagonist.

Accordingly, the invention provides a method for the treatment of a human HSV infection comprising administering a patient in need thereof with an effective amount of an agent selected from the group consisting of:
a) an antagonist US11/HIPK2 complex;
b) a HIPK2 agonist; and
c) a US11 antagonist.

Advantageously, said method enables for preventing replication of a human HSV in a subject or for restoring apoptosis of cells infected with a human HSV, in particular by interfering with US11 inhibition of cellular apoptosis.

The invention further relates to the use of an agent selected from the group consisting of:
a) an antagonist of US11/HIPK2 complex;
b) a HIPK2 agonist; and
c) a US11 antagonist;
for the manufacture of a medicament intended for the treatment or the prevention of a human HSV infection in a patient in need thereof.

A modulator of the US11/HIPK2 interaction and/or complex may be identified according to any of the screening methods described above. Examples of such modulators include for instance the HIPK2 PEST domain, which may act as a competitor with HIPK2 for US11 binding, as well as mutated form of US11 or HIPK2 that non longer have the capacity to bind with their interacting protein, *i.e.* HIPK2 and US11, respectively. Such mutated forms may readily be identified by the one skilled in the art by performing random mutations in the US11 or HIPK2 gene, especially in that portion of the gene involved in protein interaction, and assessing the capacity of the resulting mutated proteins to interact with either native US11 or HIPK2, where appropriate.

*"HIPK2 agonist"* is intended for any compound capable of promoting HIPK2 *in vitro* or *in vivo* activity, in particular induction of cell apoptosis following UV exposure. Examples of HIPK2 agonists include a nucleic acid sequence encoding a HIPK2 protein, or a HIPK2 protein, for instance.

*"US11 antagonist"* is intended for any compound capable of inhibiting US11 *in vitro* or *in vivo* activity, in particular rescue from UV-induced cell apoptosis. Examples of US11 antagonists may include antibody directed against US11, antisense nucleic acid inhibiting US11 expression, RNAi, or a ribozyme.

An *"antisense nucleic acid"* is a single stranded nucleic acid molecule which, on hybridizing under cytoplasmic conditions with complementary bases in an RNA or DNA molecule, inhibits the latter's role. If the RNA is a messenger RNA transcript, the antisense nucleic acid is a countertranscript or mRNA-interfering complementary nucleic acid. As presently used, "antisense" broadly includes RNA-RNA interactions, RNA-DNA interactions, ribozymes and RNase-H mediated arrest. Antisense nucleic acid molecules can be encoded by a recombinant gene for expression in a cell (e.g., U.S. Patent No. 5,814,500 ; U.S. Patent No. 5,811,234), or alternatively they can be prepared synthetically (e.g., U.S. Patent No. 5,780,607).

RNA interference (*RNAi*) technology prevents the expression of genes by using small RNA molecules such as « small interfering RNAs" (siRNAs). This technology in turn takes advantage of the fact that RNAi is a natural biological mechanism for silencing genes in most cells of many living organisms, from plants to insects to mammals (Sharp, 2001). RNAi would prevent a gene from producing a functional protein by ensuring that the molecule intermediate, the messenger RNA copy of the gene is destroyed. siRNAs could be used in a naked form and incorporated in a vector, as described above.

In the above therapeutic methods or applications, the therapeutic agent may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Such pharmaceutical compositions containing a therapeutic agent alone or in combination with another active ingredient may thus be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono-or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidity and/or dissolve in the rectal cavity to release the drug.

A therapeutic agent may be administered in any of the foregoing compositions and according to dosage regimens established in the art, for instance whenever specific blockade of the US11 or HIPK2 activity is required.

The daily dosage of the products may be varied over a wide range from 0.01 to 1,000 mg per adult human per day. For oral administration, the compositions are preferably provided in the form of tablets containing 0.01, 0.05, 0.1, 0.5, 1.0, 2.5, 5.0, 10.0, 15.0, 25.0, 50.0, 100, 250 and 500 milligrams of the active ingredient for the symptomatic adjustment of the dosage to the patient to be treated. A medicament typically contains from about 0.01 mg to about 500 mg of the active ingredient, preferably, from about 1 mg to about 100 mg of active ingredient. An effective amount of the drug is ordinarily supplied at a dosage level of from about 0.0002 mg/kg to about 20 mg/kg of body weight per day. Preferably, the range is from about 0.001 to 10 mg/kg of body weight per day, and especially from about 0.001 mg/kg to 7 mg/kg of body weight per day. The compounds may be administered on a regimen of 1 to 4 times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The invention will be further illustrated by the following figures and examples.

### FIGURES

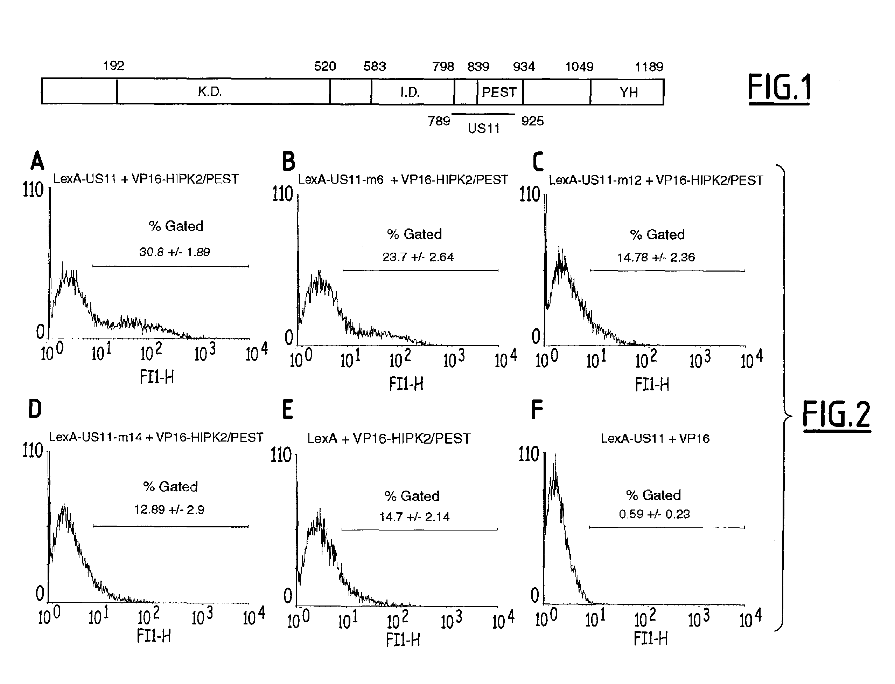

Figure 1 is a schematic representation of the HIPK2 coding sequence. KD: kinase domain; ID: interaction domain for homeoproteins, PEST: proline, glutamic acid, serine and threonine rich domain, YH: region rich in tyrosine and histidine amino acids. Numbers indicate amino acids position. Black bar: interacting region of HIPK2 with US11.
Figure 2 represents a flow cytometry analysis of the interaction of VP16-HIPK2/PEST with LexA-US11 and LexA-US11 mutant forms. Protein/protein interaction measured by the yeast two hybrid analyses using the *GFP* gene as a reporter. Yeast strain YRN974 with an integrated LexA-operator-GFP gene was used for these analyses. **A-** Yeast cells were transformed with expression vectors encoding LexA-US11 and VP16-HIPK2/PEST proteins. **B-D** Yeast cells were transformed with expression vectors encoding VP16-HIPK2/PEST combined with various vectors coding for different LexA-US11 mutants, *i.e.* LexA-US11-m6, LexA-US11-m12, LexA-US11-m14. Mutations carried by these proteins are detailed in (25). **E**- Yeast cells expressing LexA alone and VP-16-HIPK2/PEST. **F**- Yeast cells expressing LexA-US11 and VP-16 alone.

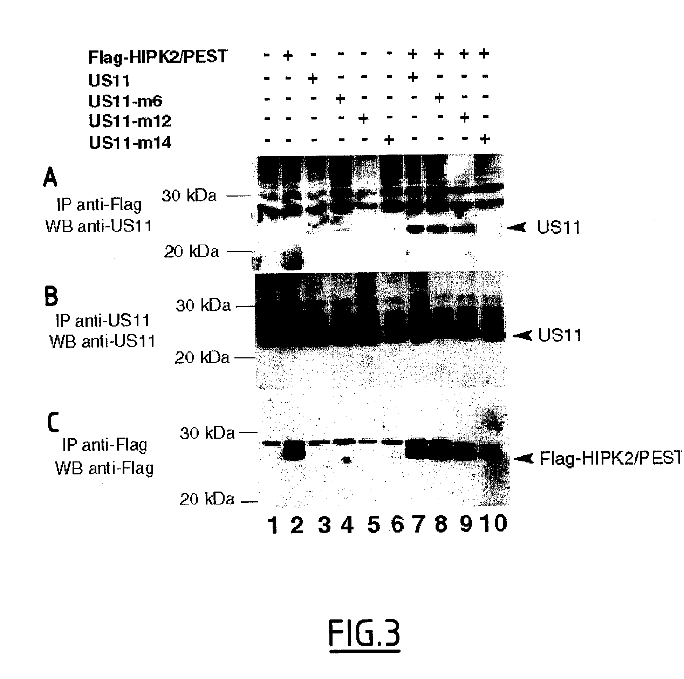

Figure 3 shows the characterization of the HIPK2 PEST domain interaction with US11 and US11 mutants in Human cells. HeLa cells were cotranfected with empty vectors (lane 1) or the indicated expression vectors containing a cDNA encoding Flag-HIPK2/PEST (lane 2, 7-10), US11 (lane 3 and 7), US11-m6 (lane 4 and 8), US11-m12 (lane 5 and 9), US11-m14 (lane 6 and 10). Cell lysates were subjected to immunoprecipitation (IP) with an anti-Flag Ab **(A** and **C)** or an anti-US11 Ab **(B).** Purified proteins were analyzed by western blot using an anti-US11 **(A** and **B)** or an anti-Flag **(C).** Arrowhead indicates US11 **(A** and **B)** or Flag-HIPK2/PEST (C). Molecular sizes are shown on the left in kDa.

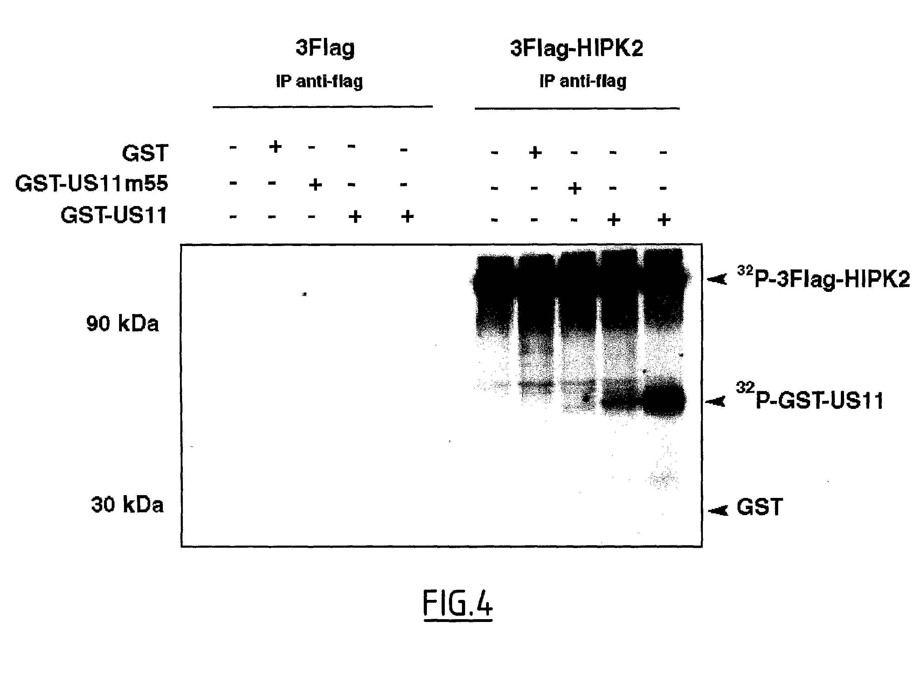

Figure 4 shows the *in vitro* phosphorylation of US11 by HIPK2. Cell were transfected with the 3Flag and 3Flag-HIPK2 expression vectors, and immunoprecipitation was carried out with an anti-Flag antibody. Kinase activity was further assayed on the immunoprecipitated protein in presence of a GST-US11 protein or a phosphorylation mutant thereof, GST-US11m55 (Catez et al. 2002) *i.e.* a US11 protein mutated in the phosphorylation site, as a negative control.

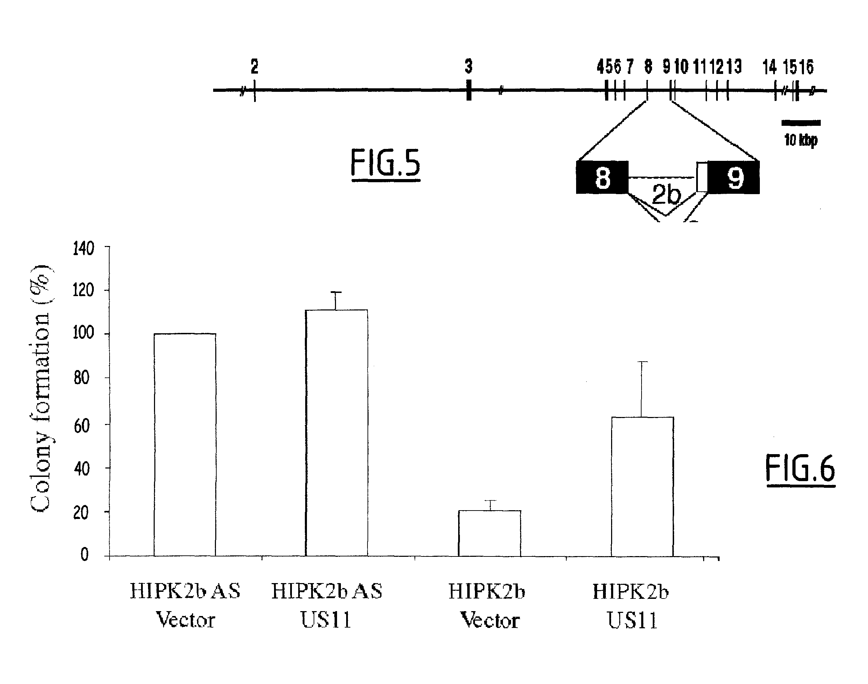

Figure 5 represents the structural organization of the murine HIPK2 gene. The bioinformatic analysis is described in Experimental procedures. Exons are represented by filled boxes. Hatch marks upstream of exon 2, in the intron3' of exon 2 and 15 indicate regions for which a portion of the intron is unknown. Alternative splicing occuring between exons 8/9 are indexed 2a and 2b.
Figure 6 is a graphical representation of antagonizing effect of US11 expression towards HIPK2 cell growth arrest. Hek 293 cells were transfected with plasmids p3flag-HIPK2b, p3flag-HIPK2b AS combined with either expression vector for US11 or the corresponding empty vector. 24 hours after transfection, cells were selected with G418 and surviving colonies were stained with cristal violet. Colony formation with the control vector p3flag-HIPK2b AS was set as 100%. Results are mean ± s.d. for three independant experiments.

### EXAMPLES

### Example 1: Experimental procedures

### Cell Culture and transfection

HeLa cells were grown in Eagle's essential minimum medium supplemented with 5% FCS and 2mM glutamine. Hek 293 cells were maintained in DMEM supplemented with 10% FCS and 2mM glutamine. Transfection of HeLa cells were carried out using the calcium phosphate precipitation procedure. The cells were plated on 35 mm diameter Petri dishes (10⁵ cells/dish) and incubated at 37°C for 24 hours. One hour prior to transfection, the medium was replaced by 1 ml of fresh medium. The transfection mix containing 1.5 µg of plasmid DNA was added to the cells. Experiments were performed 24 hours after transfection.

### Antibodies

Antibodies used for western blot analysis, immunoprecipitation and immunofluorescence include: anti-Flag M2 (Sigma-Aldrich), rabbit polyclonal anti-US11 (24) and Texas Red labeled anti-rabbit IgG (Jackson ImmunoResearch).

### Yeast Two-hybrid system

Plasmids, *S*. *cerevisiae* strains, selective media and the transformation protocol have already been described (Vojtek et al., 1995). For construction of the Lex-A-wild type US11 bait, the cDNA encoding the entire US11 sequence (Diaz et al., 1996) was inserted into the BTM116 vector, resulting in production of a Lex-A-wild type US11 fusion protein. The S. *cerevisiae* L40 strain was transformed and selected in a medium lacking uracil and tryptophan. Selected colonies were tested for the expression of the Lex-A-wild type US11 fusion protein. For this analysis yeast lysates were made using glass beads in Laemmli buffer (Laemmli et al., 1970) and expression of the Lex-A-wild type US11 protein was analyzed by western blot using an anti-US11 antibody. A colony containing the Lex-A-wild type US11 bait was then transformed with the VP16 mouse embryonic days 9.5 and 10.5 cDNA library. Transformants were selected on medium lacking uracil, tryptophan, leucine and histidine. Colonies containing a VP16 fusion protein interacting with the bait were selected on the basis of transactivation of the *His3* reporter gene after 3 days of growth. Purified DNA isolated from positives clones was amplified by PCR using VP16-specific sense and antisense primers (Vojtek et al., 1995) and analyzed by enzymatic digestion and sequencing. Sequence comparisons were done with the BLAST program (http://www.ncbi.nlm.nih.gov).

For quantification of the interaction and test of the US11 mutant (US11mt) baits the *S*. *Cerevisiae* strain YRN974 with an integrated Lex-A-operator GFP cassette was used (yEGFP3, (Cormack et al., 1996 ; Cormack et al., 1997). Ten thousand cells of four independent transformants were analyzed for fluorescence intensity using a Becton Dickinson FACScan flow cytometer (Niedenthal et al., 1996).

### Molecular cloning of HIPK2a and HIPK2b complete CDS and Plasmid Constructions

The sequence corresponding to the complete CDS of HIPK2 protein was cloned by two sets of RT-PCR performed with OF-1 Swiss adult heart mouse total mRNA. The primers used were designed according to the sequence of Kim et al., 1998. The first 5' half part of the sequence was obtained using primer 149: 5'-TACGAAGGTATGGCCTCA-3' (SEQ ID No: 13) and 1964: ATTGGTAGTTTAGTATGCAGAC-3' (SEQ ID No: 14). The second part of the sequence was cloned using primers 1648: 5'-AAAGAAGATGCTGACCATCG-3' (SEQ ID No: 15) and 3723: 5'-AAACATATGGGCCTCCTCCAGTGTTTATA-3' (SEQ ID No: 16). Both fragments were separately cloned in the pGEM-T easy vector (Promega) giving constructs pGEM-T1 (insert 149-1964) and pGEM-T3 (insert 1648-3723). Sequencing analysis revealed that 50% of the cloned 1648-3723 inserts contained a deletion of 81 nucleotides compared to the sequence published previously (3045). Plasmids carrying these shorter 1648-3723 inserts were named pGEM-T5. The complete CDS were then reconstituted by insertion of the *Cla*l/*Nsi*l fragments of pGEM-T3 or pGEM-T5 in pGEM-T1 giving constructs pGEM-HIPK2a and pGEM-HIPK2b respectively. These two plasmids were sequenced and accession numbers (available from EMBL/Genbank/DDBJ) for HIPK2a and HIPK2b sequences reported in this application are AF333791 and AF333792 respectively.
- pEGFP-HIPK2a and pEGFP-HIPK2b expression vectors:
   The pEGFP-HIPK2a and pEGFP-HIPK2b plasmids were obtained by inserting the *Eco*RI Klenow-filled fragment of constructs pGEM-HIPK2a and pGEM-HIPK2b into the *Sma*l site of pEGFP-C3 expression vector (Clontech). These two constructs were then digested by *Hind*III and recircularized to clone HIPK2a and HIPK2b CDS in frame with that of the EGFP. These constructions were confirmed by nucleotide sequencing.
- US11 and US11 mutants vectors:
   The US11 expression vector named pG-9, containing the US11 coding sequence placed under the control of CMV immediate-early- promotor and the corresponding control vector pG-8 in which the US11 coding sequence was removed were previously cloned in our laboratory (Besse et al., 1996). Construction of the expression plasmids named, pG61-US11-m6, pG61-US11-m12, pG61-US11-m14 coding for mutated forms of the US11 protein, are described in details elsewhere (Catez et al., 2002).
- pSG5-Flag-HIPK2/PEST expression vector:
   The Flag-M2 was inserted in the *EcoR*I/*Bgl*II sites of the pSG5 vector (Stratagene). The full length sequence contained in a positive two-hybrid VP16 clone was amplified by PCR using oligonucleotides 5'-GAGTTTGAGCAGATGTTTA-3' (SEQ ID No: 17) and 5'-GGAAGATCTGTGAATTCG-3' (SEQ ID No: 18) which contained a *Bgl*II site (underlined). This amplified fragment was then submitted to restriction digestion using *BamH*I and *Bg*/II enzymes and inserted in the pSG5-flag construct giving the pSG5-Flag-HIPK2/PEST plasmid.
- p3Flag-HIPK2b and p3Flag-HIPK2b AS expression vector:
   These expression vectors were obtained by inserting the *E*coRI fragment of constructs pGEM-HIPK2b into the *E*coRI site of plasmid 3XFLAG-CMV-10 (Sigma). This allowed to obtain the HIPK2b sequence cloned in both orientations, giving the plasmid p3flag-HIPK2b which contains the HIPK2b cDNA in the sense orientation and the plasmid p3flag-HIPK2b AS which contains the HIPK2b cDNA in the antisense orientation.

### Western blot analysis and Immunoprecipitation

Cells were harvested 48 h after the onset of transfection as follows: they were scraped in phosphate-buffered saline and pelleted by centrifugation. The cell pellet was resuspended in 1.5 ml of PBS and divided in three equivalent fractions. After centrifugation, cells from 2 fractions were lysed in 400 µl of RIPA buffer for immunoprecipitation (50 mM Tris-HCI pH 7.2, 1% NP40, 1% deoxycholate, 0.1% SDS and 150 mM NaCl, supplemented with protease inhibitor (Complete^{TM}, Roche molecular biochemicals)). Cells from the third fraction were resuspended in Laemmli buffer without glycerol and bromophenol blue to allow quantification of proteins. Proteins (10 µg) from total cell lysates were separated by 1D-PAGE in a gel containing 15% polyacrylamide and transferred to a PVDF membrane (Immobilon-P, Millipore).

Proteins of interest were identified by incubating the membranes with primary antibodies (anti-Flag M2 or anti-US11) in TBS-T buffer (Tris-HCl 20 mM pH 7.4, NaCl 130 mM, 0.1% Tween 20). The immunoblots were revealed by ECL (Amersham^{TM}) after incubation with a peroxidase-labelled secondary antibody. For immunoprecipitation, cell extracts were incubated at 4°C for 30 min in RIPA buffer and centrifugated at 75000 rpm (TLA 100.3 rotor) in a TL-100 Beckman centrifuge for 30 min at 4°C. Supernatants were then incubated with the anti-Flag M2 or the anti US11 overnight at 4°C. Immunocomplexes were immobilized on protein-A sepharose beads (Amerham Pharmacia Biotech, CL4B) by a 30 min incubation at 4°C. The beads were collected and washed 3 times with RIPA buffer. Bound proteins were eluted and analyzed by western blotting using the same antibodies.

### Immunohistochemistry and indirect immunofluorescence

HeLa cells were transfected with the combinations of the following expression vectors as indicated on the figures: pEGFP-HIPK2a, pEGFP-HIPK2b, pG-9, pG61-US11-m6, pG61-US11-m12, pG61-US11-m14. Cells were fixed with 3% paraformaldehyde in PBS for 30 min at room temperature and washed with a 10 mM glycine-PBS solution. They were then permeabilized for 5 min with a solution of 1% Triton X-100 in PBS. After a 10 mM glycine-PBS washing, cells were incubated in a 25 mM glycine-PBS solution for 30 min. They were incubated with an anti-US11 antibody overnight, washed and incubated with a secondary anti-rabbit IgG Ab conjugated with Texas Red. Epi-fluorescence was visualized using an Axiophot microscope from Zeiss coupled with a CCD camera (LH 750, Lhesa electronic) at excitation wawelengths of 489 nm for GFP and 596 nm for Texas Red.

### Semi-quantitative RT-PCR

Mouse total RNA collection (Ambion™, Clontech) were reverse transcribed and specific RNA were amplified using one-step RT-PCR kit (Qiagen). HIPK2a, HIPK2b mRNAs and 18S rRNA were reverse transcribed and amplified in the same reaction using primers: 18S rRNA sense 5'-ATGCGGCGGCGTTATTC-3' (SEQ ID No: 19) and 18S antisense 5'-GCGCGGGCGGTGTGTA-3' (SEQ ID No: 20); HIPK2a/HIPK2b mRNA sense 5'-GTCACCATGACACACCTGCT-3' (SEQ ID No: 21) antisense 5'-AGGGGGACACACGATGAGAG-3' (SEQ ID No: 22). PCR products were separated by agarose gel electrophoresis and quantified using a gel analyzer (Bio-1D Vilbert Lourmat) after ethidium bromide staining.

### Colony formation assay

Cells plated in 60 mm diameter Petri dishes were transfected using the calcium phosphate method with different combinations of the following plasmids as indicated on the figures: p3Flag-HIPK2b, p3Flag-HIPK2b AS; pG-9 (US11 expression vector) and pG-8 (empty vector). Total amount of transfected DNA was 4.5 µg. At 24 hours after transfection, cells were cultivated in medium containing 1 mg/mL of G418 (Geneticin, an analogue of gentamicin) for 2 weeks before staining of the surviving colonies with crystal violet. Colonies were counted using lmagemaster 2D (Pharmacia™).

### Theoretical structure of the mouse HIPK2 gene

Mouse genomic draft DNA sequences (accession number AC069142 and AC079129) homologous to HIPK2a and HIPK2b cDNAs were found using the BLAST program. Unordered pieces were ordered using the SIM4 program (Florea et al., 1998) to draw the murine HIPK2 gene structure.

### Example 2 : Results

### US11 protein interacts specifically with the PEST domain of HIPK2

A yeast two-hybrid system was used to identify cellular proteins that could interact with HSV-1 US11 protein. In this system, the 'bait protein', LexA-US11, was a fusion protein made of the DNA binding domain of the LexA operator and of the entire US11 protein. The 'prey proteins' were fusion proteins made of the activator domain of VP16 and of fragments of proteins of about one hundred aminoacids long derived from a mouse embryo cDNA library (Vojtek et al., 1995).

Using this system, twenty million of prey fusion proteins have been tested for their ability to interact with US11 protein. Among those, four hundred were shown to interact with US11 protein and forty one out of the four hundred were identified as fusion proteins containing the region encompassing amino acids 789 to 925 of HIPK2.

As shown in figure 1 this region overlaps with the PEST domain of HIPK2 (amino acids 839-934) and a nuclear speckle retention signal (SRS, amino acids 860-967). However, because US11 protein and the PEST domain of HIPK2 both exhibited a high proline content, and because this kind of domains may interact to each other non specifically (Williamson et al., 1994) it was determined whether single or a few aminoacids substitutions within US11 sequence could affect its ability to interact with VP16-HIPK2/PEST.

Three expression vectors coding for modified forms of the LexA-US11 wild type protein, designated LexA-US11-m6, LexA-US11-m12 and LexA-US11-m14 were constructed (Catez et al., (2002) for a detailed description of these modified forms) and individually transfected together with that coding for VP16-HIPK2/PEST in the yeast strain YRN974. In this strain, the reporter gene encodes the green fluorescent protein (GFP) under the control of a LexA-binding-site containing promoter, and the efficiency of the interaction between fusion proteins is estimated by numbering the fluorescent cells together with measuring the intensity of their fluorescence with a flow cytometer.

LexA-US11, but also LexA-US11-m6 interacted strongly with VP16-HIPK2/PEST since 31 and 23.7 % respectively of the cells displayed a very high level of fluorescence (Fig. 2A and B). Conversely, the interaction of LexA-US11-m12 and LexA-US11-m14 with VP16-HIPK2/PEST was greatly impaired since only 14.78 and 12.89 % of the cells displayed a high level of fluorescence (Fig. 2C and D), a percentage very close to that obtained when expressing only the LexA part of the fusion protein (Fig. 2E). The percentage of highly fluorescent cells was also very low when expressing LexA-US11 together with the DNA binding domain of VP16 only (Fig. 2F).

Therefore these results lead to the conclusion that the interaction observed between LexA-US11 and VP16-HIPK2/PEST occurred indeed via the US11 and HIPK2 parts of the fusion proteins.

To firmly validate these results, it has been further determined whether a specific interaction between the native form of US11 protein and the PEST domain of HIPK2 could be obtained after their synthesis in eukaryotic cells (Fig. 3).

An expression vector coding for a Flag-tagged HIPK2/PEST domain was constructed and co-transfected in HeLa cells, either alone or together with expression vectors coding for US11, US11-m6, US11-m12 and US11-m14. Forty height hours after transfection, proteins interacting with Flag-HIPK2/PEST were purified by co-immunoprecipitation using an anti-Flag antibody and analyzed by western blot using an anti-US11 protein antibody (Fig. 3).

US11 and US11-m6 proteins were efficiently co-immunoprecipitated with Flag-HIPK2/PEST (Fig. 3A, lane 7 and 8), whereas co-immunoprecipitation of US11-m12 was much less efficient (Fig. 3A, lane 9). US11-m14 was never co-immunoprecipitated with Flag-HIPK2/PEST (Fig. 3A, lane 10) even though both proteins, Flag-HIPK2/PEST and US11-m14, were effectively synthesized (Fig. 3B and C, lane 10). Because US11-m6 and US11-m14 exhibit respectively only one or two proline residue substitution out of thirty-five present in US11 and because their ability to interact with the PEST domain of HIPK2 is very different it can be concluded that the overall proline content of the two proteins does not account for their interaction.

Moreover, the results obtained for US11-m12 in which only an arginine residue has been replaced by a glutamine clearly demonstrated that the interaction of US11 with HIPK2 could be greatly impaired without changing the proline content of both proteins.

Therefore, all together these results demonstrate that US11 protein interacts specifically with the PEST domain of HIPK2.

The inventors further showed that US11 is phosphorylated by HIPK2 in vitro (Fig. 4). Since US11 has been shown to be phosphorylated *in vivo,* in particular by a cell kinase, this result suggests that HIPK2 may be the kinase responsible for US11 phosphorylation *in vivo.*

### Structural organization of HIPK2 gene and mRNA expression

To analyze further the interaction between US11 and HIPK2 an experimental strategy to isolate by RT-PCR the entire coding sequence of HIPK2 was designed. This experimental strategy described in details in the materials and methods section allowed to isolate and to characterize two slightly different HIPK2 cDNAs from the heart of adult Swiss mouse. The deduced sequences from these two cDNAs were designated HIPK2a and HIPK2b (GenBank accession number AF333791 and AF333792 respectively).

The major difference between these cDNAs is an 81 bp deletion within HIPK2b corresponding to the sequence coding for the domain of HIPK2 that was shown to interact with the homeoproteins (Kim et al., 1998). A BLAST analysis indicated that the longer cDNA (HIPK2b) coded for the HIPK2 protein identified previously in mice (Kim et al., 1998) also named NBAK1b or STANK (AF170302 and AF273680 respectively). The sequence of the shorter one (HIPK2a) corresponded to that of nuclear body associated kinase1a (NBAK1a, AF170301), also named PKM and identified previously in hamster (Trost et al., 2000). The sequence of HIPK2a displays a twenty-seven amino acids deletion in the region of the molecule initially described by Kim et al. (1998) as containing the domain interacting with homeoproteins. This difference might result from an alternative splicing of HIPK2 pre-mRNA.

To test this possibility, the theoretical structure of the HIPK2 gene was elucidated to determine whether potential alternative splicing sites are found within this gene. For this, large fragments of murine genomic DNA encompassing HIPK2 sequences were found in Genbank and contiguously ordered according to the HIPK2 coding sequence (see the materials and methods section).

This analysis indicated that the murine HIPK2 gene, located on chromosome 6B (Hofmann et al., 2000), is composed of at least 16 exons and lies into a chromosomal fragment longer than 150 kbp (Fig. 5). Several sequences are still missing however to reconstitute the entire sequence of the gene: sequences localized before exon 2 coding for the 5' untranslated region and therefore encompassing exon 1 and intron 1; sequences localized in introns 3 and 14 as well as those coding for the 3' untranslated region of the mRNA. This HIPK2 gene model correlated very well with the model predicted by the LocusLink software (http://www.ncbi.nlm.nih.gov/LocusLink). Sequences of every intron/exon junction enabled to validate the intron/exon arrangement proposed for the HIPK2 gene since all of these sequences are in very good agreement with those known to be potential splicing site (Sharp et al., 1987). Finally, the presence of one splicing donor site at the boundary of exon/intron 8, followed respectively by two different acceptor splicing sites at the boundary of intron8/exon9 supports the hypothesis that HIPK2a and 2b derived from a differently spliced mRNA (Fig. 5).

It was then determined whether both mRNAs coding for either HIPK2a or HIPK2b were present in mice embryos and into different tissues of adult mice and estimated their relative amounts. For this, fragments of DNA corresponding specifically to either HIPK2a or HIPK2b and to the 18S ribosomal RNAs (rRNAs) were obtained by simultaneous amplification by RT-PCR using total RNAs extracted from embryos and from various mice tissues. DNAs were separated by electrophoresis and stained with ethidium bromide. The ratios between the amount of DNA obtained from 18S rRNA and that obtained from both HIPK2 mRNAs were calculated.

This analysis showed that the two forms of HIPK2 mRNAs were expressed in all the tissues analyzed (testis, embryo, ovary, thymus, spleen, liver, brain, lung and heart). Furthermore this analysis showed that both mRNA are generally present in approximately equal amount except for the kidney and the brain in which HIPK2b mRNA is respectively the major and the minor form.

### Sub-cellular distribution of HIPK2a and HIPK2b

The cDNAs coding for HIPK2a and 2b being cloned, the intracellular distribution of both proteins was then analyzed. Two expression vectors coding for fusion proteins made of the 'enhanced green fluorescent protein' (EGFP) and of either HIPK2a or HIPK2b were constructed and transfected into HeLa cells.

Forty-eight hours after transfection, both proteins display an identical intracellular distribution, present into numerous nuclear dots, excluded from nucleoli and almost undetectable in the cytoplasm. This intracellular distribution is in good agreement with that already described for HIPK2 (Kim et al., 1998 ; Trost et al., 2000). Moreover, several reports demonstrated that HIPK2 can also be recruited to ND10.

Thus it was determined whether HIPK2 co-localized with the protein PML, one of the major component of ND10. To this aim, PML was visualized by immunofluorescence using an anti-PML antibody (kindly provided by Dr R. van Driel, Swammerdam Inst. For Life Sciences, Amsterdam, the Netherlands) in cells transiently expressing EGFP-HIPK2a and the fine localization of both proteins was determined by confocal microscopy. Numerous small nuclear dots contained only one of the two proteins, whereas other dots contained both proteins.

These findings are also in good agreement with previous studies which reported that HIPK2 localizes to distinct sub-cellular structures, *i.e.* ND10 and other nuclear speckles of unknown function (Kim et al., 1998 ; Trost et al., 2000 ; Hofmann et al., 2002 ; d'Orazi et al., 2002).

The above results demonstrated that US11 protein of HSV-1 interacts with the PEST domain of HIPK2 which is a common domain to HIPK2a and HIPK2b. This domain encompasses an SRS ('Speckle Retention Signal') sequence. Accordingly it was investigated whether the interaction between US11 and HIPK2a or HIPK2b can disturb the sub-cellular localisation of one of them.

### Sub-cellular distribution of HIPK2a and HIPK2b when co-expressed with US11

To gain insights into the functional significance of the interaction between HIPK and US11, it was thus investigated whether the intracellular distribution of one of these proteins might be affected after co-synthesis of the other.

For this, HeLa cells were transfected with expression vectors coding for either EGFP-HIPK2a or EGFP-HIPK2b together with a vector coding for wild type US11 protein. At different times after transfection, expression of the transgenes was monitored by direct visualization of EGFP fusion proteins and by immunofluorescence for US11 protein.

These experiments demonstrated that at each time analyzed after transfection, the typical pattern of intracellular distribution of each protein was dramatically different when proteins were synthesized together. For example, at 48 hours after transfection, HIPK2a and HIPK2b did not display a fine punctuate nuclear pattern but rather were found within nuclear structures that spread into the nuclei and tend to occupy the entire space, always excluding however, nucleoli. Another striking feature of US11-induced HIPK2 pattern was the presence within the cytoplasm of dots containing HIPK2. The usual nuclear distribution of US11 protein described many times (Puvion-Dutilleul, 1987 ; Roller et al., 1992) - concentrated into nucleoli and almost undetectable in the nucleoplasm - was also modified by co-expression of *HIPK2* and *US11* genes since in these conditions, US11 protein was detectable in regions surrounding nucleoli and spread throughout the nucleoplasm. The same modifications of intracellular distribution of both proteins were also observed when Flag-HIPK2 instead of EGFP-HIPK2 fusion proteins were used.

To evaluate whether its interaction with US11 protein could account for its 'unusual' behavior, the intracellular distribution of EGFP-HIPK2b was determined after co-expression with vectors coding for modified forms of US11 that were shown to interact differently with the PEST domain of HIPK2 (Fig. 1). Interestingly, US11-m6, which interacts with the PEST domain of HIPK2 with the same efficiency than wild type US11 protein, did induce a modification of intracellular distribution of HIPK2 very similar to that induced by US11 wild type protein. Conversely, US11-m12, which interacts with the PEST domain of HIPK2 less efficiently than US11 wild type protein, and US11-m14, which does not interact with this domain, did not significantly alter this pattern.

All together, these results demonstrate that US11 protein induces a severe alteration in the intracellular distribution of HIPK2 and strongly suggest that this effect is mediated through US11 protein-HIPK2 interaction.

HIPKs are SUMOlated proteins that interact with the SUMO conjugating enzyme Ubc9. The SUMOlation of HIPKs conditioned the partitioning of HIPKs within the nuclear speckles. Interaction between HIPK2 and Ubc9 occurs via a region contained within the PEST sequence (Kim et al., 1999) which perfectly matches with the interaction domain of HIPK2 for US11. Different hypothesis thus come out. By interacting with the PEST domain of HIPK2, US11 can modify its SUMOlation by Ubc9 and thus disturb its nuclear localization. This would provide a modification of the ratio between un-SUMOlated and SUMOlated HIPKs thus modifying the proportion of nuclear matrix associated HIPKs (SUMOlated form) versus the soluble non-SUMOlated form of the protein. This possibility is conceivable since the inventors have further found that US11 interacts with Ubc9 in the double hybrid system. Moreover, PEST sequences are predicted to regulate the instability of proteins (Rogers et al., 1986). Therefore an another possibility is that by interacting with the PEST domain of HIPK2 proteins US11 might antagonizes with their degradation by an unknown mechanism. Indeed, whether the degradation of HIPKs occurs via the proteasome or another pathway still remains to be determined.

### Inhibition of HIPK2-induced cell cycle arrest by US11

It has been demonstrated that in cells containing wild-type p53, HIPK2 induces apoptosis which can be objectivized by a strong decrease of cell proliferation using colony formation assays (Hofmann et al., 2002 ; d'Orazi et al., 2002 ; Kim et al., 2002). Moreover, cells expressing US11 were shown to exhibit an increased survival capacity to thermal stress compared to that of cells that did not expressed this gene ((Diaz-Latoud et al., 1997).

To further investigate the functional relevance of US11/HIPK2 interactions colony formation assays were performed. The p53 wild type expressing cells Hek 293, were transfected with Flag-HIPK2b expressing vectors allowing also the expression of the Neo^{R} gene together with expression vectors coding for US11 or in absence of this latter. As a negative control, Hek 293 cells were transfected with a vector encoding an antisense mRNA of HIPK2b (HIPK2b AS) with or without US11 expression vectors. Twenty-four hours after the beginning of the transfection, cells were selected for resistance to G418 during 2 weeks and the number of colonies was estimated for each experimental condition (Fig. 6).

HIPK2b was shown to prevent cell growth since the number of G418 resistant colonies was reduced to 17% of those obtained after transfection of the negative control vectors HIPK2b AS alone and HIPK2b AS with the US11 expressing vector (Fig. 6). Conversely, when US11 was co-expressed with HIPK2b it strongly impaired growth arrest induced by HIPK2b since the number of colonies in these conditions represented 60% of those obtained after the transfection of the control vectors. This result clearly indicated that US11 impaired HIPK2 growth arrest.

Viruses depend on host cells for their replication and have developed different strategies to affect various signalling pathways and counteract apoptosis. Particularly, HSV-1 gene expression, DNA replication and encapsidation, all take place within nuclei into globular domains designated replication compartments (Quinlan et al., 1984 ; Liptak et al., 1996 ; Lukonis et al., 1996 ; Lukonis et al., 1997). Formation of these replication compartments requires a profound re-organization of pre-existing nuclear domains of the infected cell which proceeds into several sequential steps. Among the nuclear domains which are extensively modified during HSV-1 infection, ND10 is one of the major target. Many studies have shown that early during infection HSV-1 disrupts ND10 by inducing degradation of one of the major component of this domain, namely the PML protein (see Everett et al., 1999 for review). Moreover, ND10 has been implicated in transcriptional and apoptotic pathways regulation. Indeed, ND10 contains several proteins involved in these later processes, like p53, PML, pRB, CBP, HIPK2 and TRADD (Morgan et al., 2002 ; Matera et al., 1999).

The colony formation assay reported above clearly demonstrated that US11 antagonizes the HIPK2 inhibition of proliferation in Hek 293 cells. Since HIPK2 is known to be activated in response to irradiation by UV and to trigger p53 induced apoptosis (Hofmann et al., 2002 ; d'Orazi et al., 2002 ; Kim et al., 2002), the inventors postulate that in cells submitted to UV irradiation, US11 interfere with the HIPK2 induced apoptosis, thus allowing HSV-1 to replicate.

In conclusion, the findings reported here identify US11 as a new candidate for HSV-1 inhibition of cellular apoptosis and provides a new pathway of HSV-1 induced nuclear modification that occurs with the targeting by the virus of a nuclear domain component.

### REFERENCES

- Barbas CF, Bain JD, Hoekstra DM, Lerner RA. (1992), Semisynthetic combinatorial antibody libraries: a chemical solution to the diversity problem. PNAS USA, 89, 4457-4461
- Bhat, R. A., and Thimmapaya, B. *Proc. Natl. Acad. Sci. USA* **80,** 4789-4793 (1983)
- Benoist and Chambon, Nature 1981,290:304-310
- Besse, S., Diaz, J.-J., Pichard, E., Kindbeiter, K., Madjar, J.-J., and Puvion-Dutilleul, F. (1996) *Chromosoma* **104,** 434-444
- Brinster et al., Nature 1982, 296:39-42
- Brown, S. M., and Harland, J. (1987) *JGV* **68**, 1-18
- Cassady, K. A., Gross, M., and Roizman, B. (1998) *Journal of Virology* ***72,*** 8620-8626
- Catez, F., Erard, M., Schaerer-Uthurralt, N., Kindbeiter, K., Madjar, J.-J., and Diaz, J.-J. (2002) *Mol Cell Biol* **22**, 1126-1139
- Choi, C. Y., Kim, Y. H., Kwon, H. J., and Kim, Y. (1999) *J Biol Chem* **274,** 33194-33197
- Cormack, B. P., Valdivia, R. H., and Falkow, S. (1996) *Gene* **173**, 33-38
- Cormack, B. P., Bertram, G., Egerton, M., Gow, N. A., Falkow, S., and Brown, A. J. (1997) *Microbiology* **143**, 303-311
- DeBoer, et al., Proc. Natl. Acad. Sci. USA 1983, 80:21-25
- Diaz, J.-J., Simonin, D., Massé, T., Deviller, P., Kindbeiter, K., Denoroy, L., and Madjar, J.-J. (1993) *J Gen Virol* **74***,* 397-406
- Diaz, J.-J., Duc Dodon, M., Schaerer-Uthurralt, N., Simonin, D., Kindbeiter, K., Gazzolo, L., and Madjar, J.-J. (1996) *Nature* **379**, 273-277
- Diaz-Latoud, C., Diaz, J.-J., Fabre-Jonca, N., Kindbeiter, K., Madjar, J.-J., and Arrigo, A.-P. (1997) *Cell Stress and Chaperones* **2,** 119-131
- Duc Dodon, M., Mikaelian, I., Sergeant, A., and Gazzolo, L. (2000) *Virology* **270,** 43-53
- Everett, R. D., Freemont, P., Saitoh, H., Dasso, M., Orr, A., Kathoria, M., and Parkinson, J. (1998) *J Virol* ***72****,* 6581-6591
- Everett, R. D. (1999) *Trends Biochem Sci* **24**, 293-295
- Florea, L., Hartzell, G., Zhang, Z., Rubin, G. M., and Miller, W. (1998) *Genome Res* **8**, 967-974
- Gressner, A. M., and Wool, I. G. (1974) *J. Biol. Chem.* **249,** 6917-6925
- Harlow E. et al., Antibodies: A Laboratory Manual, Cold Spring Harbor Laboratory, New York, (1988)
- Hofmann, T. G., Mincheva, A., Lichter, P., Droge, W., and Lienhard Schmitz, M. (2000) *Biochimie* **82**, 1123-1127
- Hofmann, T. G., Moller, A., Sirma, H., Zentgraf, H., Taya, Y., Droge, W., Will, H., and Schmitz, M. L. (2002) *Nat Cell Biol* **4**, 1-10
- Jayasena S.D. (1999) Aptamers: an emerging class of molecules that rival antibodies in diagnostics. Clin Chem. 45(9):1628-50
- Kim, Y. H., Choi, C. Y., Lee, S. J., Conti, M. A., and Kim, Y. (1998) *J Biol Chem* **273,** 25875-25879
- Kim, Y. H., Choi, C. Y., and Kim, Y. (1999) *Proc Natl Acad Sci U S A* **96,** 12350-12355.
- Kim, E. J., Park, J. S., and Um, S. J. (2002) *J Biol Chem* **29,** 29
- Kohler and Milstein (1975) Continuous cultures of fused cells secreting antibody of predefined specificity. Nature, 256, 495-7.
- Kollias et al., Ce II 1986, 46: 89-94
- Koshizuka, T., Takakuwa, H., Goshima, F., Murata, T., and Nishiyama, Y. (2001) *Biochem Biophys Res Commun* **288**, 597-602
- Laemmli, U. K. (1970) *Nature* **227**, 680-685
- Leopardi, R., Van Sant, C., and Roizman, B. (1997) *Proc Natl Acad Sci U S A* **94**, 7891-7896
- Li, X., Wang, Y., Debatin, K. M., and Hug, H. (2000) *Biochem Biophys Res* Common **277,** 513-517
- Liptak, L. M., Uprichard, S. L., and Knipe, D. M. (1996) *J Virol* **70,** 1759-1767
- Longnecker, R., and Roizman, B. (1986) *Journal of Virology* ***58,*** 583-591
- Lukonis, C. J., and Weller, S. K. (1996) *J Virol* **70**, 1751-1758
- Lukonis, C. J., Burkham, J., and Weller, S. K. (1997) *J Virol* **71**, 4771-4781
- MacLean, C. A., Rixon, F. J., and Marsden, H. S. (1987) *JGV* **68**, 1921-1937
- Maouche et al., Blood 1991, 15:2557
- Matera, A. G. (1999) *Trends Cell Biol* **9,** 302-309
- Matsuo, R., Ochiai, W., Nakashima, K., and Taga, T. (2001) *J Immunol Methods* **247**, 141-151
- Mogram et al., Nature 1985,315:338-340
- Morgan, M., Thorburn, J., Pandolfi, P. P., and Thorburn, A. (2002) *J Cell Biol* **157**, 975-984
- Niedenthal, R. K., Riles, L., Johnston, M., and Hegemann, J. H. (1996) *Yeast* **12**, 773-786
- Nishiyama, Y., Kurachi, R., Daikoku, T., and Umene, K. (1993) *Virology* **194,** 419-423
- D'Orazi, G., Cecchinelli, B., Bruno, T., Manni, I., Higashimoto, Y., Saito, S., Gostissa, M., Coen, S., Marchetti, A., Del Sal, G., Piaggio, G., Fanciulli, M., Appella, E., and Soddu, S. (2002) *Nat Cell Biol* **4**, 11-19
- Pierantoni, G. M., Fedele, M., Pentimalli, F., Benvenuto, G., Pero, R., Viglietto, G., Santoro, M., Chiariotti, L., and Fusco, A. (2001) *Oncogene* **20**, 6132-6141
- Poppers, J., Mulvey, M., Khoo, D., and Mohr, I. (2000) *J Virol* **74,** 11215-11221
- Puvion-Dutilleul, F. (1987) *EJCB* **43,** 487-498
- Quinlan, M. P., Chen, L. B., and Knipe, D. M. (1984) *Cell* **36,** 857-868.
- Reeck et al., Cell 50:667, 1987
- Rogers, S., Wells, R., and Rechsteiner, M. (1986) *Science* **234**, 364-368
- Roller, R. J., and Roizman, B. (1992) *Journal of Virology* **66**, 3624-3632
- Roller, R. J., Monk, L. L., Stuart, D., and Roizman, B. (1996) *Journal of Virology* **70**, 2842-2851
- Sharp, P. A. (1987) Science 235, 766-771
- Sharp, Genes Dev, 2001, 15:485-490
- Trost, M., Kochs, G., and Haller, O. (2000) *J Biol Chem* **275**, 7373-7377
- Tuerk C. and Gold L. (1990) Systematic evolution of ligands by exponential enrichment: RNA ligands to bacteriophage T4 DNA polymerase. Science. 3;249(4968):505-10
- Villa-Komaroff, et al., Proc. Natl. Acad. Sci. USA 1978,75:3727-3731
- Vojtek, A. B., and Hollenberg, S. M. (1995) *Methods Enzymol* 255, 331-342
- Wagner et al., Proc. Natl. Acad. Sci. USA 1981, 78: 1441-1445
- Wang, W., Link, V., and Green, J. M. (2000) *Cell Immunol* **205**, 34-39
- Wang, Y., Hofmann, T. G., Runkel, L., Haaf, T., Schaller, H., Debatin, K., and Hug, H. (2001a) *Biochim Biophys Acta* **1518,** 168-172
- Wang, Y., Debatin, K. M., and Hug, H. (2001b) *BMC Mol Biol* **2,** 8
- Ward, P. L., and Roizman, B. (1994) *Trends in Genetics* **10,** 267-274
- Waterhouse P, Griffiths AD, Johnson KS, Winter G. (1993) Combinatorial infection and in vivo recombination: a strategy for making large phage antibody repertoires. Nucleic Acids Research, 21, 2265-2266
- Williamson, M. (1994) *Biochem. J*. **297,** 249-260

## Claims

1. An isolated US11/HIPK2 protein complex comprising two interacting proteins selected, said complex being from the group consisting of (a) US11 and HIPK2, (b) US11 and a fragment of HIPK2, (c) a fragment of US11 and HIPK2, and (d) a fragment of US11 and a fragment of HIPK2.

2. The protein complex according to claim 1, wherein said HIPK2 fragment comprises the PEST domain.

3. An antibody specific for the US11/HIPK2 protein complex according to claim 1 or 2.

4. A composition comprising at least two interacting proteins selected from the group consisting of (a) US11 or a fragment of US11, and (b) HIPK2 or a fragment of HIPK2

5. A kit comprising (a) US11 or a fragment of US11, and (b) HIPK2 or a fragment of HIPK2.

6. An isolated host cell that comprises a HIPK2 expressing vector and a US11 expressing vector.

7. An *in vitro* method of screening or identifying candidate molecules capable of modulating a US11/HIPK2 complex that comprises the steps consisting of:
a) contacting at least one candidate molecule with a US11/HIPK2 protein complex according to claim 1 or 2, and/or with US11 and HIPK2 proteins; and
b) determining the presence or absence of binding of the candidate molecule to said US11/HIPK2 complex, US11 protein or HIPK2 protein;
wherein the binding of the candidate molecule to said US11/HIPK2 complex, US11 protein or HIPK2 protein, is indicative of a modulator of the US11/HIPK2 complex.

8. The method according to claim 7, wherein said modulator is a modulator of the interaction of US11 with HIPK2 or of US11/HIPK2 complex activity.

9. The method according to claim 8, wherein said modulator is a modulator of US11/HIPK2 complex activity, which method comprises the steps consisting of:
a) contacting at least one candidate molecule with a US11/HIPK2 protein complex to claim 1 or 2; and
b) determining the presence or absence of binding of the candidate molecule to said US11/HIPK2 complex;
wherein the binding of the candidate molecule to said US11/HIPK2 complex is indicative of a modulator of the US11/HIPK2 complex activity.

10. A method of screening or identifying candidate molecules capable of modulating activity of a US11/HIPK2 complex that comprise the steps consisting of:
a) contacting a p53 expressing cell containing a HIPK2 expressing vector and a US11 expressing vector with a p53-mediated apoptosis inducer, in the presence or in the absence of a candidate molecule, under conditions sufficient to allow apoptosis to occur where the candidate molecule is absent; and
b) determining the number of cell colonies in the presence and in the absence of the candidate molecule;
wherein an altered number of colonies as estimated in the presence of the candidate molecule, compared with the number of colonies as estimated in the absence of the candidate molecule, is indicative of a molecule capable of modulating activity of a US11/HIPK2 complex.

11. An *in vitro* method for a method of screening or identifying candidate molecules capable of modulating the US11 and HIPK2 interaction, said method comprising the steps consisting of:
a) contacting at least one candidate molecule with a HIPK2 protein and a US11 protein, under conditions suitable to allow US11 and HIPK2 to interact in the absence of the candidate molecule; and
b) determining the interaction of US11 and HIPK2 in the absence and in the presence of the candidate molecule;
wherein a modulation of the interaction of US11 and HIPK2 is indicative of a molecule capable of modulating the US11 and HIPK2 interaction.

12. The method according to claim 11, wherein said molecule capable of modulating the US11 and HIPK2 interaction is an antagonist and wherein a decreased interaction of US11 and HIPK2 as measured in the presence of the candidate molecule, compared with US11 and HIPK2 interaction as measured in the absence of the candidate molecule, is indicative of an antagonist of the US11 and HIPK2 interaction.

13. The method according to claim 11, wherein said molecule capable of modulating the US11 and HIPK2 interaction is an agonist and wherein a increased interaction of US11 and HIPK2 as measured in the presence of the candidate molecule, compared with US11 and HIPK2 interaction as measured in the absence of the candidate molecule, is indicative of an agonist of the US11 and HIPK2 interaction.

14. The method according to any of claims 7 to 13, for identifying molecules useful for preventing a human herpes simplex virus infection.

15. An *in vitro* method of screening molecules useful for preventing or treating a human herpes simplex virus infection, wherein a candidate molecule is assayed for its capacity to interfere with US11 inhibition of cellular apoptosis.

16. The method according to claim 15, which comprises the steps consisting of:
a) cultivating a cell containing a HIPK2 expressing vector and a US11 expressing vector, in the presence or in the absence of a candidate molecule, under conditions sufficient to allow HIPK2 expression and cell apoptosis to occur where the candidate molecule is absent; and
b) determining the number of cell colonies in the presence and in the absence of the candidate molecule;
wherein a decreased number of colonies as estimated in the presence of the candidate molecule, compared with the number of colonies as estimated in the absence of the candidate molecule, is indicative of a molecule useful for preventing or treating a herpes simplex virus infection.

17. A composition comprising an antagonist of US11/HIPK2 complex, in a pharmaceutically acceptable carrier.

18. The composition according to claim 17, wherein said antagonist of US11/HIPK2 complex is a specific antagonist of the interaction of US11 with HIPK2.

19. The composition according to claim 17, wherein said antagonist of US11/HIPK2 complex is an antagonist of the US11/HIPK2 complex activity.

20. Use of an agent selected from the group consisting of:
a) an antagonist US11/HIPK2 complex;
b) a HIPK2 agonist; and
c) a US11 antagonist
for the manufacture of a medicament intended for the treatment of a human herpes simplex virus infection in a patient in need thereof.

21. The use according to claim 20 wherein said medicament is intended for preventing replication of a human herpes simplex virus in a subject or for restoring apoptosis of cells infected with a human herpes simplex virus.
